# EUROPÄISCHE PATENTSCHRIFT

(11) **EP 2 974 681 B1**
(45) Veröffentlichungstag und Bekanntmachung des Hinweises auf die Patenterteilung: **11.12.2019**
(21) Anmeldenummer: 15170067.1
(22) Anmeldetag: 01.06.2015
(51) Int. Cl.: B01F 11/00, B01F 13/00, B01F 15/02, B29K 33/00, A61B 17/88, B01F 15/00, A61L 24/06

(54) **VAKUUMMISCHSYSTEM UND VERFAHREN ZUM MISCHEN VON POLYMETHYLMETHACRYLAT-KNOCHENZEMENT**
VACUUM MIXING SYSTEM AND METHOD FOR MIXING OF POLYMETHYLMETHACRYLATE BONE CEMENT
SYSTÈME DE MÉLANGE SOUS VIDE ET PROCÉDÉ DE MÉLANGE DE CIMENT OSSEUX EN POLYMÉTHACRYLATE DE MÉTHYLE

(30) Priorität: 15.07.2014 DE 102014109905
(43) Veröffentlichungstag der Anmeldung: 20.01.2016
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A2- 2 730 332
- DE-U1- 20 008 103

## Beschreibung

Die Erfindung betrifft ein Vakuummischsystem zum Mischen von Polymethylmethacrylat-Zement (PMMA-Zement) aus zwei Ausgangskomponenten, insbesondere zum Mischen eines medizinischen Knochenzements, und zur Lagerung der Ausgangskomponenten.

Die Erfindung betrifft ferner ein Verfahren zum Mischen von Polymethylmethacrylat-Knochenzement.

Gegenstand der Erfindung ist somit ein Vakuummischsystem für die Lagerung, Vermischung und gegebenenfalls auch das Austragen von Polymethylmethacrylat-Knochenzement.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A. Bei den dort offenbarten Vakuum-Zementiersystemen wird zur Erzeugung des Unterdrucks eine externe Vakuumquelle angeschlossen.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden mit der EP 0 692 229 A1, der DE 10 2009 031 178 B3, der US 5,997,544 A, der US 6,709,149 B1, der DE 698 12 726 T2 und der US 5,588,745 A vorgeschlagen. Das Patent DE 10 2009 031 178 B3 und EP 2 730 332 A2 offenbaren dabei ein geschlossenes Vakuummischsystem mit einem zweiteiligen Austragskolben zum Verschluss einer Zement-Kartusche. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

Die Aufgabe der Erfindung besteht also darin, die Nachteile des Stands der Technik zu überwinden. Insbesondere soll ein einfach aufgebautes und kostengünstig zu fertigendes, geschlossenes Vakuummischsystem für Polymethylmethacrylat-Knochenzement und ein Verfahren zum Mischen eines Zwei-Komponenten-Knochenzements unter Vakuum bereitgestellt werden. Dabei soll auch eine alternative Lösung bereitgestellt werden. Bevorzugt soll das Vakuummischsystem mit möglichst wenigen beweglichen Teilen auskommen. Insbesondere soll auch ein einfacher Aufbau des Austragskolbens ermöglicht werden. Die Bedienung des Vakuummischsystems soll einfach und intuitiv sein.

Dieses Vakuummischsystem soll eine Zement-Kartusche enthalten, in der das Zementpulver gelagert ist, sowie einen davon separaten Vorratsbehälter, in dem sich die Monomerflüssigkeit befindet. Die Monomerflüssigkeit wird dadurch getrennt von dem Zementpulver gelagert. Vor und während der Vermischung der beiden Zementkomponenten, dem Zementpulver mit der Monomerflüssigkeit, soll ein Kontakt der medizinischen Anwender mit diesen Komponenten ausgeschlossen sein. Die Öffnung des Vorratsbehälters und der Transfer des Monomers müssen daher in einem geschlossenen System erfolgen. Das Zementpulver darf ebenfalls nicht in Kontakt zum medizinischen Anwender kommen. Bei einer Ethylenoxid-Sterilisation des Vakuummischsystems ist es unumgänglich, dass das Ethylenoxid in das in der Zement-Kartusche befindliche Zementpulver eindringt und anschließend wieder aus diesem austritt. Für diesen Gasaustausch ist eine Öffnung in der Kartusche zur umgebenden Atmosphäre mit einer großen Gasaustauschfläche erforderlich. Dabei darf jedoch das Zementpulver nicht aus der Zement-Kartusche austreten. Das bedeutet, das Vakuummischsystem muss so beschaffen sein, dass ein Gasaustauch möglich ist, ohne dass das Zementpulver austreten kann. Weiterhin muss die Zement-Kartusche während des Mischvorgangs vakuumdicht verschlossen werden können.

Die Erfindung hat daher ferner die Aufgabe, eine Vakuummischvorrichtung bereitzustellen, welche den Widerspruch zwischen der maximalen Gasdurchlässigkeit der Zement-Kartusche zum Gasaustausch während der Ethylenoxid-Sterilisation bei gleichzeitiger Verhinderung des Austritts des Zementpulvers und der Vakuum-Dichtigkeit der Zement-Kartusche während des Vermischungsvorganges löst. Das zu entwickelnde Vakuummischsystem soll möglichst aus üblichen thermoplastischen Kunststoffen durch Spritzgießen hergestellt werden können und damit für Einmalanwendungen geeignet sein.

Des Weiteren soll eine kostengünstig zu fertigende und zuverlässig funktionierende Vorrichtung zum Mischen eines medizinischen Zements und gegebenenfalls zur Lagerung der Ausgangskomponenten sowie ein Verfahren zum Mischen des Knochenzements gefunden werden, bei dem eine möglichst einfache Bedienung zum Mischen der Ausgangskomponenten angewendet werden kann, möglichst ohne dass Lufteinschlüsse in dem Zementteig entstehen können.

Die Hauptkomponente des Polymethylmethacrylat-Knochenzements soll ein Pulver sein und die zweite Komponente soll in Form einer Flüssigkeit vorliegen. Die beiden Ausgangskomponenten des Knochenzements sollen bevorzugt in dem Vakuummischsystem getrennt gelagert werden können und durch Anwendung der Vorrichtung sicher zusammengeführt werden können.

Die Aufgaben der Erfindung werden gelöst durch ein Vakuummischsystem zum Mischen von Polymethylmethacrylat-Knochenzement aufweisend zumindest eine Kartusche mit einem evakuierbaren Innenraum zum Mischen des Knochenzements, wobei der Innenraum einen zylindrischen Hubraum umfasst, ein Mischelement, das im Innenraum der Kartusche beweglich angeordnet ist und das von außerhalb des Vakuummischsystems zum Mischen des Inhalts im Innenraum der Kartusche bedienbar ist und einen Austragskolben mit einem zylindrischen Außenumfang, dessen erste Grundfläche eine Grundfläche des Innenraums der Kartusche begrenzt, der lösbar mit der Kartusche zu arretieren oder arretiert ist und der im gelösten Zustand beweglich im zylindrischen Bereich des Innenraums der Kartusche ist, wobei in dem Austragskolben eine gasdurchlässige und für Partikel undurchlässige Durchführung angeordnet ist oder zwischen dem Austragskolben und der Innenwand des Innenraums eine Durchführung gebildet ist, wobei die Durchführung sich von einer Öffnung in der Mantelfläche des Austragskolbens bis zu einer Öffnung in der ersten Grundfläche des Austragskolbens erstreckt.

Ein Zylinder im Sinne der vorliegenden Erfindung ist, wie auch nach der allgemeinen Definition, ein von zwei parallelen, ebenen, kongruenten Flächen (Grund- und Deckfläche) und einer Mantelfläche beziehungsweise Zylinderfläche begrenzter Körper, wobei die Mantelfläche von parallelen Geraden gebildet wird. Das heißt, der Zylinder entsteht durch Verschiebung einer ebenen Fläche entlang einer Geraden, die nicht in dieser Ebene liegt. Die Höhe des Zylinders ist gegeben durch den Abstand der beiden Ebenen, in denen Grund- und Deckfläche liegen.

Sind die Geraden senkrecht zu Grund- und Deckfläche, spricht man von einem geraden Zylinder. Die gerade zylindrische Geometrie des Innenraums ist erfindungsgemäß bevorzugt, bezieht sich aber besonders bevorzugt nur auf einen oder mehrere Teilbereich des gesamten Innenraums der Kartusche. Ein gerader Kreiszylinder stellt im Sinne der vorliegenden Erfindung also nur einen Spezialfall einer zylindrischen Geometrie dar, der aber wegen der besonders einfachen Möglichkeiten zu dessen Herstellung eine besonders bevorzugte Symmetrie ist.

Unter einem zylindrischen Außenumfang des Austragskolbens ist vorliegend zu verstehen, dass der Austragskolben zumindest in einem Abschnitt einen zylindrischen Umfang aufweist, der die maximale radiale Ausdehnung bezüglich des Austragskolbens bezogen auf die zylindrische Geometrie des zylindrischen Abschnitts bildet. Der Austragskolben kann bereichsweise auch eine andere Form als die zylindrische Form aufweisen, insbesondere können zumindest ein Dichtungsring, zumindest ein Rastmittel oder Gegenrastmittel und eine Abstreiflippe zusätzlich zu der Öffnung in der Mantelfläche vorhanden sein. Bevorzugt dichtet der Austragskolben den zylindrischen Hubraum mit dem zylindrischen Außenumfang bis auf die Durchführung und einen Vakuumanschluss ab, wenn der zylindrische Außenumfang innerhalb des Hubraums angeordnet ist.

Wenn die Durchführung zwischen dem Austragskolben und der Innenwand des Innenraums eine Durchführung gebildet ist, kann beispielsweise in dem Zylindermantel des Austragskolbens und/oder der Innenwand des Innenraums eine Rinne vorgesehen sein, die die Durchführung begrenzen. Die Durchführung bezieht sich dabei auf den an den Innenwänden des Innenraums anliegenden Austragskolben. Die Durchführung und die Öffnungen können daher auch durch die Bereiche definiert sein, an denen der Austragskolben nicht an den Innenwänden des Innenraums anliegt, was beispielsweise eben auch durch eine Vertiefung, einer Rille oder Rinne in der ansonsten zylindrischen Innenwand der Kartusche definiert werden. Bevorzugt ist die Durchführung jedoch in dem Austragskolben angeordnet.

Der Begriff Unterdruck bezieht sich vorliegend immer auf einen relativ zur umgebenden Atmosphäre bezogenen Druck, der kleiner ist als der umgebende Atmosphärendruck.

Besonders bevorzugt ist der Knochenzement ein PMMA-Knochenzement. Der Polymethylmethacrylat-Knochenzement (PMMA-Knochenzement) wird bevorzugt aus zumindest zwei Komponenten gemischt beziehungsweise ist aus zumindest zwei Komponenten herstellbar. Besonders bevorzugt ist eine Komponente flüssig und eine andere Komponente pulverförmig.

Auch im zylindrischen Bereich des Hubraums des Austragskolbens können Vertiefungen, wie eine Nut, oder Vorsprünge, wie eine Feder oder ein umlaufender Ring, angeordnet sein, so dass die zylindrische Symmetrie bereichsweise durchbrochen sein kann. Hierdurch kann in dem zylindrischen Bereich eine Rastung für ein Rastmittel am Austragskolben realisiert werden. Das Gleiche gilt dementsprechend für den zylindrischen Außenumfang des Austragskolbens.

Das Vakuummischsystem ist bevorzugt nach außen Gasdicht ausgebildet beziehungsweise gasdicht abdichtbar.

Mit einer Weiterbildung der Erfindung wird vorgeschlagen, dass der Austragskolben wenigstens eine umlaufende Dichtung aufweist, die den Innenraum der Kartusche nach außen abdichtet, wobei bevorzugt wenigstens eine umlaufende Dichtung zwischen einer zweiten Grundfläche des Austragskolbens, die der ersten Grundfläche des Austragskolbens gegenüberliegt, und der Öffnung in der Mantelfläche des Austragskolbens angeordnet ist.

Hierdurch wird eine bessere Vakuumstabilität des Innenraums erreicht. Dadurch kann der Unterdruck oder das Vakuum im Innenraum länger aufrechterhalten werden.

Es kann bei einer Ausführungsvariante der vorliegenden Erfindung vorgesehen sein, dass eine der Mantelflächen der Kartuschenwandung der Kartusche eine Öffnung aufweist, die mit der Öffnung in der Mantelfläche des Austragskolbens in einer geöffneten Position des Austragskolbens in Überdeckung liegt und durch die der Innenraum gasdurchlässig mit der Umgebung des Vakuummischsystems verbunden oder verbindbar ist.

Hierdurch kann der Austragskolben tief und stabil in der Kartusche eingesetzt bleiben. Dadurch kann eine bessere Stabilität des Vakuummischsystems erreicht werden und der Austragskolben kann weniger leicht verkanten und kann etwas kürzer ausgeführt werden.

Dabei kann vorgesehen sein, dass an der Außenwand der Kartusche ein Verschlusselement angeordnet ist, mit dem die Öffnung in der Wandung der Kartusche verschließbar ist, bevorzugt ein in axialer Richtung der Kartusche verschiebbares Verschlusselement an der Außenwand der Kartusche angeordnet ist.

Hierdurch muss die Öffnung in der Kartuschenwandung nicht vom Austragskolben oder zumindest nicht vom Austragskolben alleine verschlossen werden. Zudem zieht sich ein auf der Kartuschenwandung außen angeordnetes Verschlusselement unter der Einwirkung eines Vakuums im Inneren des Innenraums von alleine zu und dichtet die Öffnung in der Kartuschenwandung ab. Alternativ kann der Austragskolben auch so gegen die Öffnung der Kartuschenwandung verdreht und/oder verschoben werden, dass die Durchführung nicht mehr in Überdeckung mit der Öffnung in der Kartuschenwandung liegt und dadurch die Durchführung im oder am Austragskolben verschlossen ist beziehungsweise verschließbar ist.

Bei der Ausführung mit einem Verschlusselement auf der Außenwandung der Kartusche kann wiederum vorgesehen sein, dass das Verschlusselement eine umlaufende Manschette ist, die in Passung auf der Außenwand der Kartusche anliegt und in axialer Richtung der Kartusche verschiebbar ist, um die Öffnung in der Wandung der Kartusche abzudecken und dadurch zu verschließen, wobei bevorzugt an der Manschette wenigstens ein Griffstück befestigt ist, das zum manuellen Verschieben der Manschette auf der Außenwand der Kartusche vorgesehen ist.

Die Manschette muss nicht vollständig umlaufend sein, sondern kann unterbrochen sein, das heißt eine axiale Durchbrechung aufweisen. Die Manschette kann dazu als Ringsegment oder Rohrsegment ausgebildet sein, das die Außenwand der Kartusche um zumindest 50% umschließt, bevorzugt um zumindest 60% umschließt, besonders bevorzugt um zumindest 75% umschließt. Der Innendurchmesser der Manschette ist bevorzugt gleich oder kleiner dem Außendurchmesser der Kartusche. Diese Ausführung ist besonders einfach zu bedienen und kostengünstig zu realisieren.

Bei einer alternativen Ausführung der vorliegenden Erfindung kann vorgesehen sein, dass der Austragskolben in einer ersten arretierbaren Stellung aus der Kartusche herausragt, so dass die Öffnung in der Mantelfläche des Austragskolbens offen liegt und dass der Austragskolben in einer zweiten arretierbaren Stellung tiefer in dem Innenraum der Kartusche angeordnet ist, so dass die Öffnung in der Mantelfläche des Austragskolbens durch die Innenwand der Kartusche verschlossen ist.

Diese Ausführung hat den Vorteil, dass die Kartuschenwand ohne Öffnung aufgebaut werden kann.

Dabei kann vorgesehen sein, dass zwischen der Öffnung in der Mantelfläche des Austragskolbens und der zweiten Grundfläche des Austragskolbens, die der ersten Grundfläche des Austragskolbens gegenüberliegt, ein umlaufendes Dichtungselement angeordnet ist.

Hierdurch wird die Abdichtung des Innenraums der Kartusche in den verschiedenen Stellungen des Austragskolbens sichergestellt.

Bevorzugte Ausführungsformen der Erfindung können sich dadurch auszeichnen, dass in der Durchführung und/oder an der Öffnung zur Durchführung in der Mantelfläche des Austragskolbens und/oder an der Öffnung zur Durchführung in der ersten Grundfläche des Austragskolbens ein gasdurchlässiger Partikelfilter, insbesondere ein Porenfilter, angeordnet ist, wobei bevorzugt der gasdurchlässige Partikelfilter für Partikel mit einem Durchmesser von über 1 µm undurchlässig ist, besonders bevorzugt für Partikel mit einem Durchmesser von über 5 µm undurchlässig ist.

Hierdurch wird sichergestellt, dass das Zementpulver aus dem Innenraum der Kartusche in dem Innenraum verbleibt, während Gase aus dem Innenraum evakuiert werden können und andere Gase, wie beispielsweise Ethylenoxid, eingefüllt werden können.

Gemäß einer Weiterbildung der vorliegenden Erfindung wird vorgeschlagen, dass der Austragskolben an seiner aus dem Inneren der Kartusche weisenden Seite bis auf eine Vakuumdurchführung gasdicht ist.

Mit der Vakuumdurchführung, kann dann ein Vakuum im Inneren der Kartusche gezogen werden, nachdem die Durchführung verschlossen wurde.

Ferner kann vorgesehen sein, dass der Austragskolben an der ersten Grundfläche eine gasdurchlässige Porenscheibe aufweist, die durch eine Verrippung gestützt wird. Hierdurch kann die Porenscheibe mechanisch stabilisiert werden.

Gemäß einer weiteren Ausführung kann vorgesehen sein, dass der Austragskolben axial in die Kartusche eindrückbar ist, um einen aus einem Knochenzementpulver und einer Monomerflüssigkeit gemischten Knochenzementteig durch eine Austragsöffnung an einem dem Austragskolben gegenüberliegenden Ende der Kartusche auszutragen, wobei bevorzugt der Austragskolben nach dem Lösen einer Arretierung axial in die Kartusche eindrückbar ist.

Dadurch wird sichergestellt, dass der Austragskolben nach dem Sterilisieren des Inhalts des Innenraums und nach dem Mischen der Knochenzementkomponenten unter Vakuum zu seinem eigentlichen Zweck, nämlich dem Austragen des Zementteigs aus der Kartusche verwendet werden kann.

Mit der Erfindung wird auch vorgeschlagen, dass eine Austragsöffnung der Kartusche ein Anschlussmittel, insbesondere ein Anschlussgewinde, aufweist.

Besonders geeignet ist als Anschlussmittel ein Anschlussgewinde in Form eines Innengewindes oder Außengewindes. Das Anschlussgewinde kann zum einen zum Schließen eines Applikationsrohrs verwendet werden und zum anderen zum Befestigen der Kartusche an einem Standfuß oder an einem Basiselement verwendet werden.

Bevorzugt kann auch vorgesehen sein, dass das Mischelement an einer Stange angeordnet ist, die durch einen gasdichten Durchgang ins Innere der Kartusche geführt ist, und sich das Mischelement durch hineinschieben und herausziehen aus der Kartusche und durch Drehen in der Kartusche bewegen lässt, wobei bevorzugt die Stange eine Sollbruchstelle aufweist, an der die Stange, wenn sie aus der Kartusche herausgezogen ist, nahe dem Durchgang abzubrechen ist.

Mit der durchgeführten Stange kann das Mischelement besonders einfach von außen bedient werden. Bevorzugt ist die Stange durch einen gasdichten Durchgang in dem Austragskolben in das Innere der Kartusche geführt.

Bevorzugte Ausführungsformen können sich dadurch auszeichnen, dass das Vakuummischsystem einen Vorratsbehälter für eine Monomerflüssigkeit aufweist, insbesondere einen Glasbehälter für eine Monomerflüssigkeit aufweist, in der Kartusche ein Zementpulver enthalten ist, wobei ein Öffnungselement zur Öffnung des Vorratsbehälters vorgesehen ist und die Kartusche über eine Leitung mit dem geöffneten Vorratsbehälter verbunden oder verbindbar ist.

Unter dem Begriff "Öffnungselement" werden Vorrichtungen verstanden, die bei Betätigung eine Öffnung des Vorratsbehälters der Monomerflüssigkeit bewirken können. Beispielhaft seien dafür Öffnungsvorrichtungen gemäß DE 10 2010 026 497 B4 und DE 10 2010 026 496 B4 genannt.

Glasampullen haben als Vorratsbehälter den entscheidenden Vorteil, dass diese völlig diffusionsbeständig und damit undurchlässig für die Monomerflüssigkeit sind. Dadurch kann die Monomerflüssigkeit über mehrere Jahre bei Raumtemperatur ohne Verluste gelagert werden.

Dabei kann vorgesehen sein, dass das Vakuummischsystem ein Basiselement aufweist, wobei das Basiselement die Kartusche, den Vorratsbehälter und das Öffnungselement lagert.

Das Basiselement beziehungsweise der Standfuß integriert die Kartusche und weitere Bauteile, die an die Kartusche angeschlossen werden können. Zudem kann das Basiselement zur stabilen Aufstellung des Vakuummischsystems verwendet werden.

Dabei kann wiederrum vorgesehen sein, dass das Basiselement ein Koppelmittel aufweist für eine kraft- und/oder formschlüssige Verbindung mit der Kartusche, insbesondere mit dem Anschlussmittel an der Austragsöffnung der Kartusche.

Damit kann eine stabile und dichte Verbindung der Kartusche zu dem Basiselement erreicht werden.

Auch kann vorgesehen sein, dass in der Leitung ein Ventilelement angeordnet ist, das den Ausfluss der Monomerflüssigkeit aus dem Vorratsbehälter in die Kartusche steuert und/oder auslöst.

Hierdurch kann verhindert werden, dass es zu einer ungewollten und verfrühten Einmischung des Monomers beziehungsweise der zweiten Zementkomponente kommt, die zu einer chemischen Härtung des Zements in der Kartusche führen könnte und damit das Vakuummischsystem blockiert.

Erfindungsgemäße Vakuummischsysteme können sich auch dadurch auszeichnen, dass an der Kartusche ein Rastmittel und an dem Austragskolben mindestens ein Gegenrastmittel vorgesehen ist, wobei der Austragskolben mit dem Rastmittel in der Kartusche in einer Position lösbar arretiert oder lösbar arretierbar ist, oder in zumindest zwei Positionen lösbar arretierbar ist, wobei die Durchführung in einer ersten arretierten Position geöffnet ist und in einer zweiten arretierten Position gasdicht verschlossen ist.

Hierdurch kann sichergestellt werden, dass der eingerastete Austragskolben nicht ungewollt durch Einwirkung des Vakuums bewegt wird. Bei gelöstem Austragskolben kann der Austragskolben mit dem Vakuum gezielt bewegt werden. Wenn der Austragskolben in der zweiten Position arretiert ist oder der die Durchführung mit dem Verschlusselement geschlossen ist, kann das Vakuum (durch den Vakuumanschluss im Austragskolben) dazu verwendet werden, die Monomerflüssigkeit beziehungsweise die zweite flüssige Zementkomponente in die Kartusche zu saugen, damit diese dort mit dem Zementpulver mit Hilfe des Mischelements im Inneren der Kartusche gemischt werden kann.

Die der Erfindung zugrundeliegenden Aufgaben werden auch gelöst durch ein Verfahren zur Vermischung von Polymethylmethacrylat-Knochenzement in einer Kartusche eines erfindungsgemäßen Vakuummischsystems, bei dem ein durch einen Austragskolben verschlossener Innenraum einer Kartusche durch eine gasdurchlässige Durchführung im Austragskolben offenliegt, wobei Gas aus dem Innenraum evakuiert wird und der Innenraum mit einem sterilisierenden Gas gefüllt wird, wobei danach die Durchführung durch Verschieben des Austragskolbens verschlossen wird oder durch Bedienen eines Verschlusselements am Außenumfang der Kartusche verschlossen wird, danach der Innenraum über eine Vakuumdurchführung evakuiert wird und danach Ausgangskomponenten des Knochenzements mit einem Mischelement im Innenraum der Kartusche unter Vakuum gemischt werden.

Dabei kann vorgesehen sein, dass der Austragskolben nach dem Einsetzen des Austragskolbens in die Kartusche mit der Kartusche arretiert wird und/oder der Austragskolben nach dem Verschieben des Austragskolbens arretiert wird, so dass er nicht durch Einwirkung des Unterdrucks in die Kartusche gezogen wird, und die Arretierung nach dem Mischen des Knochenzements gelöst wird und der gemischte Knochenzementteig aus der Kartusche durch Vortreiben des Austragskolbens im Innenraum der Kartusche durch eine gegenüberliegende Austragsöffnung ausgetrieben wird.

Des Weiteren kann vorgesehen sein, dass ein Zementpulver in dem Innenraum der Kartusche enthalten ist, eine Monomerflüssigkeit in den Innenraum der Kartusche eingeleitet wird, bevorzugt mit dem Unterdruck in dem Innenraum der Kartusche eingesaugt wird, und die Monomerflüssigkeit mit dem Zementpulver in dem evakuierten Innenraum der Kartusche gemischt wird.

Der Erfindung liegt die überraschende Erkenntnis zugrunde, dass es durch eine Durchführung im oder am Austragskolben, der mit einer Öffnung in der Mantelfläche des Arbeitskolbens und mit einer zum Innenraum gerichteten Öffnung in der ersten Grundfläche des zylindrischen Austragskolbens verbunden ist, gelingt, den Innenraum einer Kartusche zum Mischen von Knochenzement für eine Evakuierung von außen und eine Sterilisierung mit einem Gas (wie Ethylenoxid) zugänglich zu machen und gleichzeitig durch die Möglichkeit, die Durchführung zu verschließen, den Innenraum durch eine zusätzliche Vakuumdurchführung bei umgebenden Normaldruck evakuierbar zu machen, so dass der Inhalt unter Vakuumeinwirkung gemischt werden kann. Die Öffnung zum Innenraum kann einfach durch ein Verschieben oder ein Verdrehen des Austragskolbens oder mit einem Verschlusselement an der Außenwand der Kartusche abgedichtet werden. Dadurch kann das erfindungsgemäße Vakuummischsystem besonders leicht angewendet und bedient werden, wobei der Aufbau leicht umzusetzen und kostengünstig zu realisieren ist.

Ein erfindungsgemäßes, geschlossenes Vakuummischsystem ist beispielsweise zusammengesetzt aus einer Zement-Kartusche, in dem ein Mischelement angeordnet ist, wobei das Mischelement mittels einer an einem ersten Zement-Kartuschenende heraus geführten Betätigungsstange axial bewegbar ist und das erste Zement-Kartuschenende mit einem zylinderförmigen Austragskolben verschlossen ist. Das Vakuummischsystem ist dadurch charakterisiert, dass
a) am ersten Zement-Kartuschenende mindestens eine Öffnung in der Kartuschenwand angeordnet ist,
b) an der Außenwand der Zement-Kartusche ein verschiebbares Verschlusselement angeordnet ist, das in axialer Richtung mindestens eine Länge von der axialen Länge der mindestens einen Öffnung der Kartuschenwand hat, und
c) der Austragskolben mindestens eine Öffnung in der zylinderförmigen Mantelfläche besitzt.

Die Zement-Kartusche ist eine Kartusche, die zum Lagern einer Zementkomponente und zum Mischen eines Zementteigs geeignet ist.

Die Öffnung in der Kartuschenwand kann kreisförmig, elliptisch oder rechteckig ausgebildet sein. Daneben sind alle regelmäßig und auch unregelmäßig geformten Öffnungen geeignet.

Das verschiebbare Verschlusselement ist als Hohlzylinder oder als Hohlzylinder mit einer axialen Durchbrechung ausgebildet, wobei der Hohlzylinder einen Innendurchmesser gleich oder kleiner dem Außendurchmesser der Zement-Kartusche besitzt. Das Verschlusselement kann im unverschlossenen Zustand unterhalb der mindestens einen Öffnung in der Mantelfläche der Zement-Kartusche angeordnet sein. In diesem Zustand kann Ethylenoxid durch die Öffnung in das Innere der Zement-Kartusche einströmen und nach Beendigung der Sterilisation wieder austreten. Wenn das Verschlusselement axial über die mindestens eine Öffnung geschoben wird, so dass diese mindestens eine Öffnung vollständig abgedeckt ist, dann ist der Innenraum der Zement-Kartusche gasdicht verschlossen.

Das erfindungsgemäße Vakuummischsystem kann beispielsweise dadurch charakterisiert sein, dass der zylinderförmige Austragskolben
a) an seiner Oberseite gasdicht ausgebildet ist,
b) an der Oberseite einen Vakuumanschluss besitzt,
c) an der Unterseite eine gasdurchlässige Porenscheibe besitzt, die durch eine Verrippung abgestützt wird,
d) einen Innenraum der durch die gasdichte Oberseite, der Verrippung der Porenscheibe und der inneren zylindrischen Mantelfläche gebildet wird,
e) der Vakuumanschluss mit dem Innenraum gasdurchlässig verbunden ist und dass
f) an der Mantelfläche mindestens eine Öffnung angeordnet ist, die eine axiale Länge kleiner gleich dem Abstand der gasdichten Oberseite und der Verrippung besitzt, wobei die mindestens eine Öffnung gasdurchlässig über den Innenraum mit der Porenscheibe verbunden ist.

Bevorzugt ist der Austragskolben des Vakuummischsystems derart in der Zement-Kartusche lösbar angeordnet, dass die mindestens eine Öffnung der zylindrischen Mantelfläche des Austragskolbens mindestens teilweise die mindestens eine Öffnung der Zement-Kartuschenwand überdeckt. Dadurch ist es möglich, dass während der Sterilisation mit Ethylenoxid das Gas in das Innere der Zement-Kartusche durch die übereinander liegenden Öffnungen eindringen kann. Nach Beendigung der Sterilisation kann durch diese übereinander liegenden Öffnungen das verbliebene Ethylenoxid aus der Zement-Kartusche austreten. Dabei erleichtert, die große Fläche der Porenscheibe den Gasaustritt.

Wesentlich für die Funktion eines Vakuummischsystems mit einem verschiebbaren Verschlusselement ist, dass das Verschlusselement nach Verschiebung auf das erste Zement-Kartuschenende die mindestens eine Öffnung in der Zement-Kartuschenwand vollständig abdeckt. Dadurch wird der Innenraum der Zement-Kartusche gasdicht abgeschlossen. Bei Anlegen des Vakuums an den Vakuumanschluß an der Oberseite des Austragskolbens kann dadurch der Innenraum der Zement-Kartusche unter Vakuum gesetzt werden, ohne dass Luft von außen in die Zement-Kartusche einströmen kann. Durch das Vakuum im Inneren der Zement-Kartusche wird das Verschlusselement an die Zement-Kartusche angesaugt. Dadurch wird das Verschlusselement an die Außenwandung der Zement-Kartusche angepresst und die gasdichte Abdichtung verstärkt. Alternativ kann also auch das Verschlusselement drehbar an der Außenseite der Kartusche angeordnet sein, wobei das Verschlusselement in einer ersten Stellung die Öffnung offen lässt und in einer zweiten, gedrehten Stellung die Öffnung verschließt.

Vorteilhaft ist es, wenn die Stange beziehungsweise die Betätigungsstange für das Mischelement eine Sollbruchstelle aufweist. Die Betätigungsstange ist bevorzugt durch eine gasdichten Durchführung im Austragskolben drehbar und axial in Längsrichtung beweglich geführt. Diese Sollbruchstelle ist so angeordnet, dass nach Hochziehen der Betätigungsstange in Richtung des Austragskolbens aus der Kartusche hinaus, das Mischelement an der Unterseite des Austragskolbens anliegt und die Betätigungsstange direkt an der oberen Kante des Austragskolbens abgebrochen werden kann. Dadurch ist es leicht möglich, mit den Stempeln von üblichen handbetriebenen Austragsvorrichtungen den Austragskolben axial in Richtung des Kartuschenkopfs zu bewegen, wobei der Zementteig aus der gegenüberliegenden Austragöffnung ausgepresst wird.

Ein beispielhaftes erfindungsgemäßes Vakuummischsystem ist aus einem Vorratsbehälter für die Monomerflüssigkeit, einer mit Zementpulver gefüllten Zement-Kartusche, einem Öffnungselement zur Öffnung des Vorratsbehälters und einem Basiselement aufgebaut, wobei das Basiselement die Zement-Kartusche, den Vorratsbehälter und das Öffnungselement lagert. Das bedeutet, dass das Basiselement die Zement-Kartusche, den Vorratsbehälter und das Öffnungselement miteinander verbindet.

Das Basiselement des erfindungsgemäßen Vakuummischsystems weist vorteilhaft ein Koppelmittel für eine kraft- und/oder formschlüssige Verbindung mit der Zement-Kartusche auf, insbesondere mit der Austragsöffnung der Zement-Kartusche. Das Kopplungsmittel des Basiselements kann zum Beispiel als Zylinder mit Außengewinde ausgebildet sein. Die Austragsöffnung der Zement-Kartusche kann ein Innengewinde enthalten, so dass die Zement-Kartusche auf das Außengewinde des Zylinders des Basiselements aufgeschraubt werden kann. Dadurch kann die Zement-Kartusche flüssigkeitsdicht mit dem Basiselement verbunden sein beziehungsweise werden. Das Basiselement selbst kann eine Einspritzdüse für den Eintrag der Monomerflüssigkeit in das in der Zement-Kartusche gelagerte Zementpulver enthalten.

Im Basiselement des erfindungsgemäßen Vakuummischsystems ist bevorzugt ein Leitungsmittel beziehungsweise eine Leitung angeordnet. Das Leitungsmittel verbindet den Vorratsbehälter mit der Zement-Kartusche so, dass nach der Öffnung des Vorratsbehälters durch Betätigung des Öffnungselements die Monomerflüssigkeit durch das Leitungsmittel in den Vorratsbehälter fließen kann. Bevorzugt wird die Monomerflüssigkeit durch Vakuumeinwirkung aus dem Vorratsbehälter durch das Leitungsmittel in die Zement-Kartusche gesaugt.

Ähnliche Aufbauten, wie das Abschrägen eines Bereichs an der dem Innenraum zugewandten Grundfläche des Austragskolbens oder das Anbringen eines Porenfilters in der Öffnung der Kartuschenwand sind zwar aufwendiger beziehungsweise ungünstiger in der Ausführung, realisieren aber dennoch die vorliegende Erfindung.

Im Folgenden werden weitere Ausführungsbeispiele der Erfindung anhand von zehn Abbildungen beziehungsweise Figuren erläutert, ohne jedoch dabei die Erfindung zu beschränken. Dabei zeigt:
- Figur 1:: eine perspektivische Außenansicht eines erfindungsgemäßen Vakuummischsystems mit offener Durchführung;
- Figur 2:: eine perspektivische Teilschnittansicht des Vakuummischsystems nach Figur 1 mit offener Durchführung;
- Figur 3:: eine Querschnittansicht des Vakuummischsystems nach Figur 1 und 2 mit offener Durchführung;
- Figur 4:: eine Querschnittansicht eines Ausschnitts des Vakuummischsystems nach Figur 1, 2 und 3 mit geschlossener Durchführung;
- Figur 5:: eine perspektivische Außenansicht eines alternativen erfindungsgemäßen Vakuummischsystems mit offener Durchführung;
- Figur 6:: eine perspektivische Teilschnittansicht des Vakuummischsystems nach Figur 5 mit offener Durchführung;
- Figur 7:: eine Querschnittansicht des Vakuummischsystems nach Figur 5 und 6 mit offener Durchführung;
- Figur 8:: eine Querschnittansicht eines Ausschnitts des Vakuummischsystems nach den Figuren 5 bis 7 mit offener Durchführung;
- Figur 9:: eine seitliche Teilschnittansicht des Vakuummischsystems nach den Figuren 5 bis 8 mit geschlossener Durchführung; und
- Figur 10:: eine perspektivische Teilschnittansicht des Vakuummischsystems nach den Figuren 5 bis 9 mit geschlossener Durchführung.

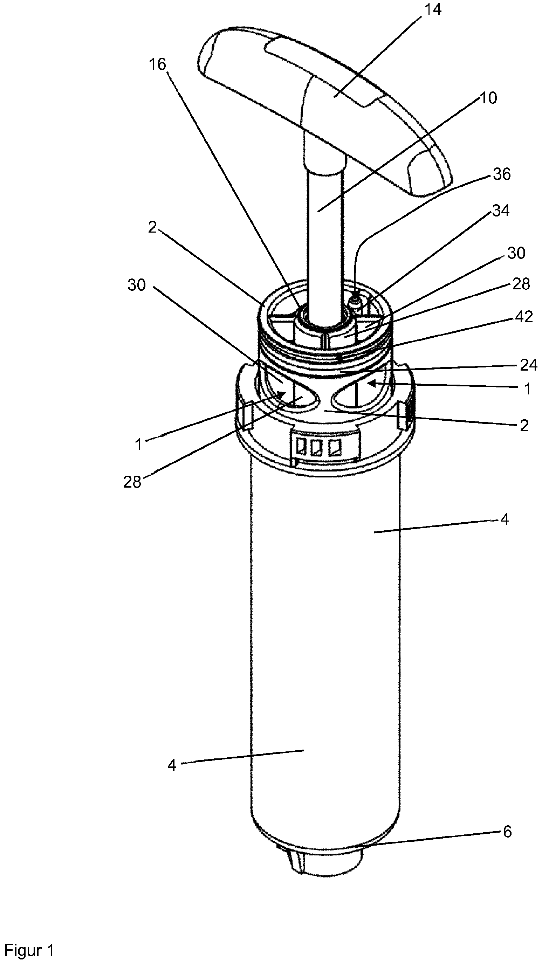

Öffnungen, Durchführungen, Innenräume und Freiräume, also nicht körperliche Teile, die nur durch eine umgebende Form definiert sind, werden in den Figuren 1 bis 10 durch Pfeile als Bezugszeichenpfeile markiert, während alle körperlichen Bauteile und Merkmale der Vakuummischsysteme nach den Figuren 1 bis 10 durch Bezugszeichenlinien gekennzeichnet werden, die an den jeweiligen Bauteilen beziehungsweise Merkmalen enden.

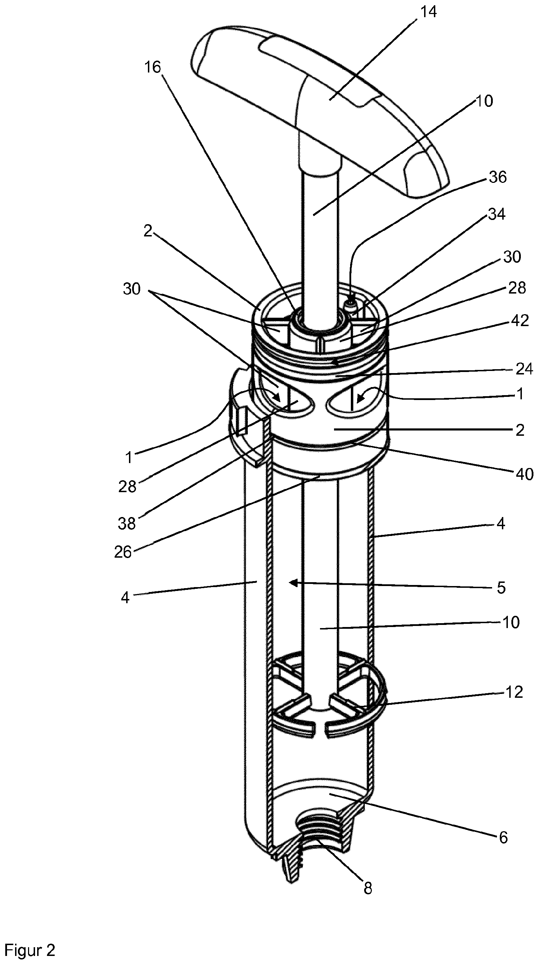

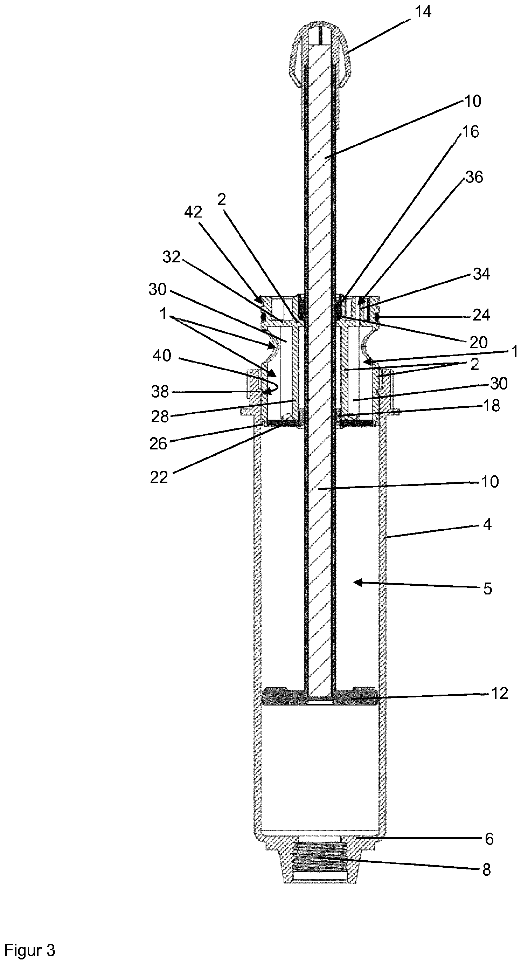

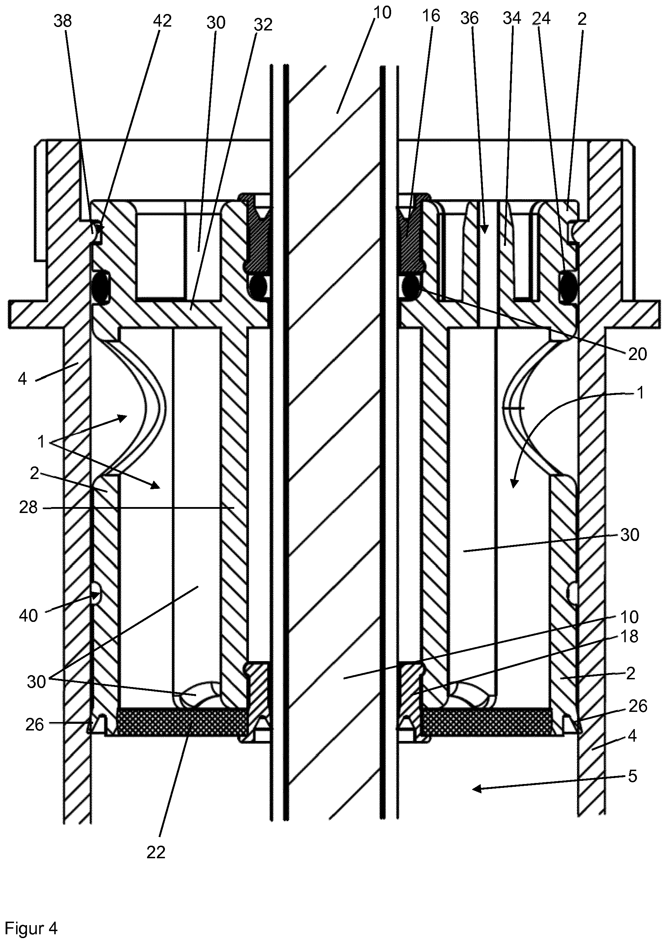

Die Figuren 1 bis 3 zeigen ein erstes Ausführungsbeispiel der vorliegenden Erfindung beziehungsweise ein erstes erfindungsgemäßes Vakuummischsystem, bei dem eine Durchführung 1 durch einen weitgehend zylindrischen Austragskolben 2 offen ist, und die Figur 4 das gleiche Ausführungsbeispiel der vorliegenden Erfindung beziehungsweise das erste erfindungsgemäße Vakuummischsystem bei dem die Durchführung 1 durch den Austragskolben 2 geschlossen ist. Dabei zeigt Figur 1 eine perspektivische Außenansicht, Figur 2 eine perspektivische Teilschnittansicht, Figur 3 eine Querschnittansicht und Figur 4 eine Querschnittansicht eines Ausschnitts des erfindungsgemäßen Vakuummischsystems.

Das Vakuummischsystem weist eine großenteils rohrförmige Kartusche 4 mit einem Innenraum 5 auf. In dem Innenraum 5 ist ein Zementpulver (nicht gezeigt) zur Herstellung eines Zementteigs aus zwei Komponenten enthalten. Die Kartusche 4 ist an der Vorderseite (in den Figuren 1 bis 3 unten und in Figur 4 nicht zu sehen) mit einer Platte 6 verschlossen, in der eine Öffnung mit einem Innengewinde 8 vorgesehen ist. An das Innengewinde 8 kann eine Basisplatte (nicht gezeigt) beziehungsweise ein Standfuß (nicht gezeigt) oder ein Austragsrohr (nicht gezeigt) zum Applizieren des fertig gemischten Zementteigs montiert werden. Über die Basisplatte beziehungsweise den Standfuß wird ein Flüssigkeitsbehälter (nicht gezeigt) mit einem flüssigen Monomer als zweiter flüssiger Komponente des Knochenzements mit der Kartusche 4 über eine Leitung (nicht gezeigt) verbunden. Dazu mündet die Leitung in einen Stutzen an der Basisplatte beziehungsweise dem Standfuß, wobei an dem Stutzen ein Außengewinde vorgesehen ist, so dass die Kartusche 4 mit dem Innengewinde 8 auf den Stutzen geschraubt werden kann beziehungsweise geschraubt ist, so dass die Leitung von dem Flüssigkeitsbehälter druckdicht über die Leitung mit der Kartusche 4 verbindbar oder verbunden ist. Ein Vakuummischsystem mit einem solchen Standfuß beziehungsweise einer solchen Basisplatte, einer solchen Leitung, einem solchen Stutzen und einem solchen Flüssigkeitsbehälter ist beispielsweise aus der DE 10 2009 031 178 B3 beziehungsweise der US 8,757,866 B2 bekannt, so dass für Details hierzu auf diese Patente verwiesen werden kann. Für die folgende Betrachtung sei angenommen, dass die Kartusche 4 an der Vorderseite zunächst nach außen abgeschlossen ist, indem die Kartusche 4 mit dem Innengewinde 8 auf einen solchen Standfuß beziehungsweise eine solche Basisplatte des Vakuummischsystems aufgeschraubt ist.

Auf der Rückseite (in den Figuren 1 bis 4 in Richtung oben) ist die Kartusche 4 durch den Austragskolben 2 verschlossen. Durch eine Durchführung im Austragskolben 2 erstreckt sich eine Stange 10, die an der Vorderseite im Innenraum 5 der Kartusche 4 in einem Mischelement 12 endet, das vier sich in Richtung der Wandung der Kartusche 4 radial ersteckenden Mischflügel aufweist. Die Stange 10 endet an der Rückseite (in den Figuren 1 bis 3 oben und in Figur 4 nicht zu sehen) in einem Handgriff 14, mit dem die Stange 10 manuell bewegt werden kann. Die Stange 10 ist um ihre Achse drehbar und in axialer Richtung (in Längsrichtung verschiebbar) in dem Austragskolben 2 gelagert. Hierzu sind in dem Austragskolben 2 zwei Lagerringe 16, 18 vorgesehen, die in Passung an der Stange 10 anliegen und die Stange 10 lagern.

Der untere Lagerring 18 dient auch der Abdichtung des Innenraums 5, um ein Austreten von Pulver oder Zementteig aus dem Innenraum 5 zu verhindern. Um den Innenraum 5 gasdicht und druckdicht abzudichten, ist zudem eine Dichtung 20 in Form eines O-Rings aus Gummi vorgesehen.

Durch die manuelle Drehbarkeit und axiale Verschiebbarkeit der Stange 10 kann der Inhalt des Innenraums 5 mit dem Mischelement 12 manuell durchmischt werden.

Die Durchführung 1 durch den Austragskolben 2 ist an der Vorderseite des Austragskolbens 2 mit einer Porenscheibe 22 abgedeckt. Die Porenscheibe 22 verhindert ein Eindringen von Zementpulver in die Durchführung 1 beziehungsweise ein Austreten von Zementpulver aus dem Vakuummischsystem.

Am Außenumfang des Austragskolbens 2 ist eine umlaufende Dichtung 24 in Form eines O-Rings aus Gummi vorgesehen, mit der der Zwischenraum zwischen dem Austragskolben 2 und der Innenwand der Kartusche 4 abgedichtet ist und damit der Innenraum 5 nach außen gasdicht und druckdicht abgeschlossen werden kann, wenn der Austragskolben 2 tief genug in die Kartusche 4 eingedrückt beziehungsweise eingeschoben ist, wie dies beispielsweise in Figur 4 gezeigt ist.

An der Vorderseite weist der Austragskolben 2 eine umlaufende Abstreiflippe 26 auf, mit der beim Austragen eines fertig gemischten Knochenzements aus dem Innenraum 5 durch die Öffnung in der Platte 6 mit Hilfe des Austragskolbens 2 der Zementteig vollständig nach vorne getrieben wird, ohne dass sich der Zementteig an der Abstreiflippe 26 vorbei aus dem Innenraum 5 herausdrücken kann. Dazu ist die Abstreiflippe 26 verformt. Die Abstreiflippe 26 ragt dabei nicht, wie in Figur 4 dargestellt, in die Wandung der Kartusche 4 hinein, sondern wird von ihrer Ausgangsform, wie sie in Figur 4 dargestellt ist, entsprechend verformt. Durch die dabei auftretende elastische Kraft wird der Innenraum 5 mit der Abstreiflippe 26 abgedichtet. Das gleiche Prinzip der Abdichtung, in dem Fall gegen die Stange 10, wird auch für den unteren Lagerring 18 angewendet.

Im Austragskolben 2 ist die Stange 10 durch eine Hülse 28 des Austragskolbens 2 geführt. Von der Hülse 28 erstrecken sich radial vier Streben 30 als Teil des Austragskolbens 2 in Richtung des Außenumfangs des Austragskolbens 2. Die Streben 30 dienen der mechanischen Stabilisierung und Formgebung des Austragskolbens 2 und der Lagerung der Porenscheibe 22. Rückseitig ist der Austragskolben 2 zwischen den Öffnungen in der Mantelfläche des Austragskolbens 2 für die Durchführung 1 und dem rückseitigen Ende des Austragskolbens 2 (in den Figuren 1 bis 4 oben) durch eine Platte 32 verschlossen. Die Platte 32 ist Teil des Austragskolbens 2 und mit diesem einteilig ausgeführt.

Eine Vakuumdurchführung 34 ist in der Platte 32 vorgesehen, die in einen Vakuumanschluss 36 in Form eines Stutzens 36 mündet. Auf den Vakuumanschluss 36 kann ein Schlauch (nicht gezeigt) aufgesteckt werden, der an eine Vakuumquelle (nicht gezeigt) anschließbar ist.

Im Innenumfang der Kartusche 4 ist eine umlaufende Feder 38 in Form eines Rings 38 vorgesehen, die als Rastmittel 38 zur Arretierung des Austragskolbens 2 in der Kartusche 4 vorgesehen ist. Als Gegenrastmittel 40, 42 sind in dem Austragskolben 2 zwei umlaufende Nuten 40, 42 beziehungsweise umlaufende Vertiefungen 40, 42 vorgesehen, so dass der Austragskolben 2 in zwei verschiedenen Positionen bezüglich seiner Bewegbarkeit in Längsrichtung in der Kartusche 4 arretierbar ist. Anstatt des Systems aus Nut 40, 42 und Feder 38 könnten auch andere, beispielsweise manuell verformbare und damit einfacher lösbare Arretierungsmittel vorgesehen sein, ohne dass hierdurch die Erfindung eingeschränkt würde.

In die an der vorderen Platte 6 geschlossene Kartusche 4 wird zunächst das Knochenzementpulver eingefüllt. Dazu ist der Austragskolben 2 zunächst noch nicht in die Kartusche 4 eingesetzt. Anschließend wird der Austragskolben 2 in die Kartusche 4 eingesteckt, bis die Feder 38 mit der unteren Nut 40 arretiert. Diese erste arretierte Position des Austragskolbens 2 ist in den Figuren 1 bis 3 dargestellt. Durch die offene Durchführung 1 kann die gesamte Kartusche 4 beziehungsweise das gesamte Vakuummischsystem in einer Kammer durch Evakuieren der Luft aus der Umgebung der Kartusche 4 beziehungsweise des Vakuummischsystems und durch Befüllen der Umgebung der Kartusche 4 beziehungsweise des Vakuummischsystems mit Ethylenoxid desinfiziert beziehungsweise sterilisiert werden.

Anschließend wird der Austragskolben 2 tiefer in die Kartusche 4 eingeschoben, bis die Feder 38 in die obere Nut 42 greift und der Austragskolben 2 in einer zweiten Position arretiert wird. Die zweite arretierte Position des Austragskolbens 2 in der Kartusche 4 ist in Figur 4 dargestellt. Durch die Innenwandung der Kartusche 4 werden die Öffnungen im Zylindermantel des Austragskolbens 2 zu den Durchgängen 1 in der zweiten Position verschlossen. Durch die Dichtung 24 wird der Innenraum 5 bis auf die Vakuumdurchführung 36 druckdicht und gasdicht verschlossen, indem die Dichtung 24 an den Innenwänden der Kartusche 4 anliegt.

An den Vakuumanschluss 34 wird ein Schlauch angeschlossen, der mit einer Vakuumquelle verbunden ist. Durch die Vakuumdurchführung 36 wird die Luft aus dem Innenraum 5 herausgesaugt. Mit der Porenscheibe 22 wird verhindert, dass Pulver aus dem Innenraum 5 in das Vakuumsystem gelangen kann. Da der Innenraum 5 bis auf die an der Platte 6 beziehungsweise dem Innengewinde 8 angeschlossene Leitung und den Flüssigkeitsbehälter druckdicht ist, wird der Innenraum 5 evakuiert. Die Leitung und der Flüssigkeitsbehälter des Vakuummischsystems sind nach außen gasdicht und druckdicht geschlossen. Durch die Leitung wird das Monomer aus dem Flüssigkeitsbehälter in den Innenraum 5 gesaugt. Dort mischt es sich mit dem Zementpu lver.

Das Zementpulver kann mit der Monomer-Flüssigkeit mit dem Mischelement 12 im Innenraum 5 unter Vakuum durch drehen und hineinstoßen und herausziehen der Stange 10 manuell gemischt werden. Nach erfolgter Mischung wird die Kartusche 4 von dem Standfuß beziehungsweise der Basisplatte abgeschraubt und auf das Innengewinde 8 wird ein Austragsrohr (nicht gezeigt) aufgeschraubt. Das Austragsrohr kann einen statischen Mischer enthalten. Die Stange 10 kann bis zum Anschlag aus dem Innenraum 5 herausgezogen werden, so dass das Mischelement 12 an der Vorderseite des Austragskolbens 2 anliegt. Die Stange 10 kann mit dem Handgriff 14 an einer Sollbruchstelle (nicht gezeigt) abgebrochen werden. Anschließend kann die Kartusche 4 in eine Auspressvorrichtung (nicht gezeigt) zum Vortreiben des Austragskolbens 2 in der Kartusche 4 eingesetzt werden, oder der Austragskolben 2 wird mit Hilfe eines Stößels (nicht gezeigt) vorgetrieben. Beispielsweise kann der Austragskolben 2 aus seiner zweiten Position entrastet werden und über das weiter anliegende Vakuum wird der Austragskolben 2 in der Kartusche 4 vorangetrieben, da außerhalb der Kartusche 4 nun kein Vakuum sondern Umgebungsdruck anliegt.

Durch das Vortreiben des Austragskolbens 2 wird der Zementteig aus dem Innenraum 5 der Kartusche 4 herausgetrieben und über das Austragsrohr appliziert.

Alle Teile des Vakuummischsystems sind aus Kunststoff durch Spritzguss gefertigt, wobei die Dichtungen 20, 24 bevorzugt aus Gummi oder einem anderen elastischen Kunststoff bestehen.

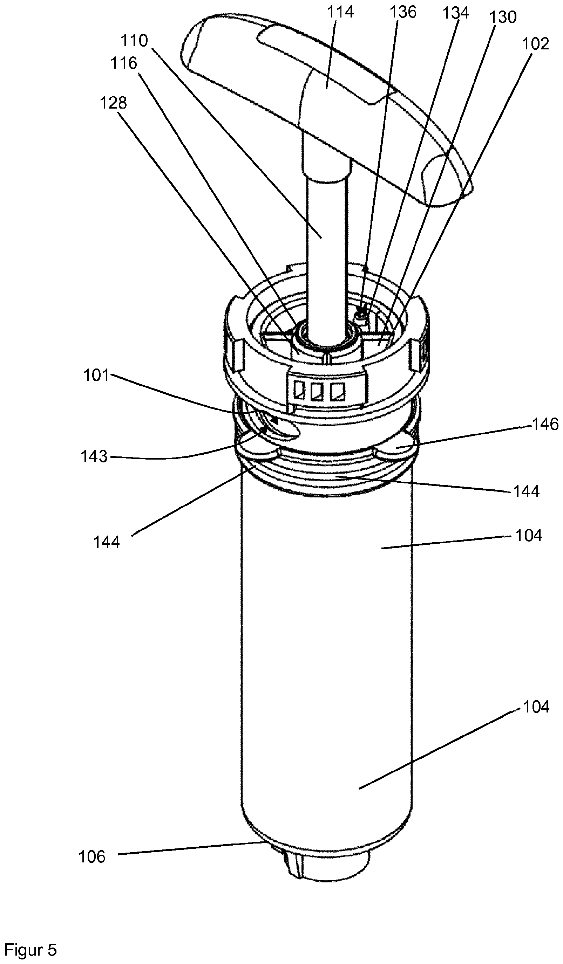

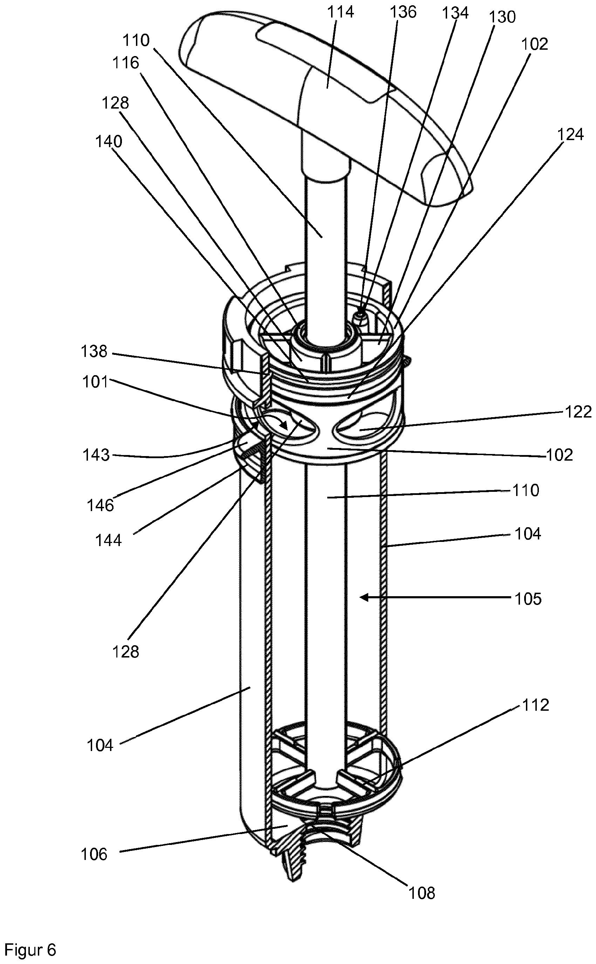

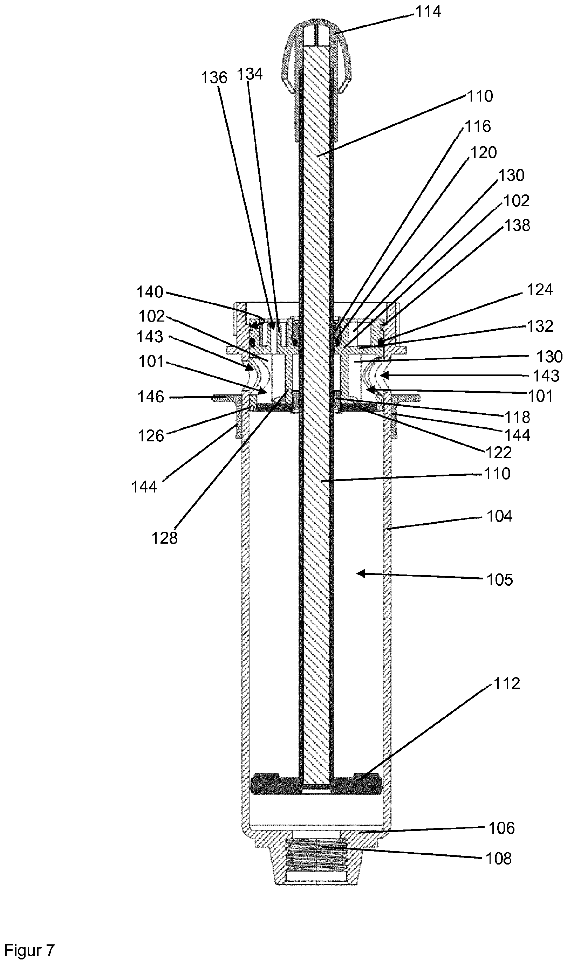

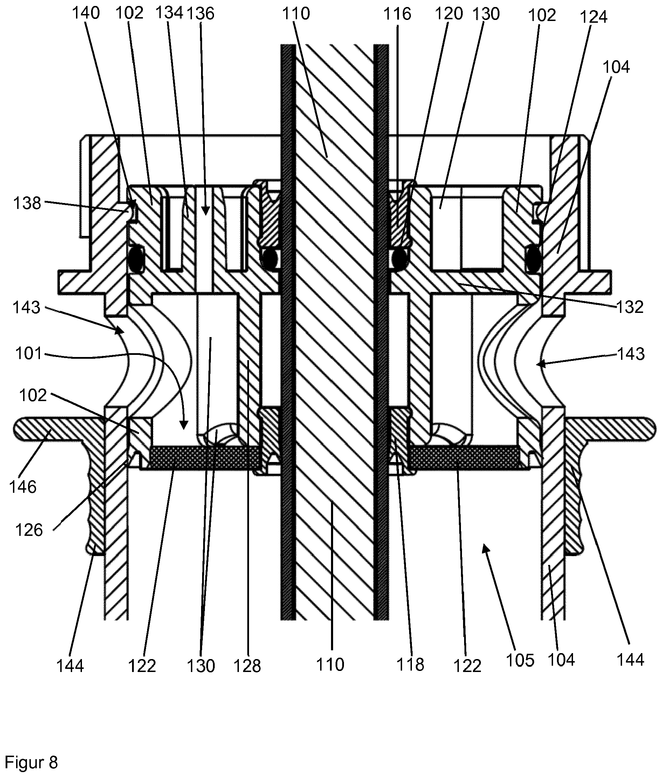

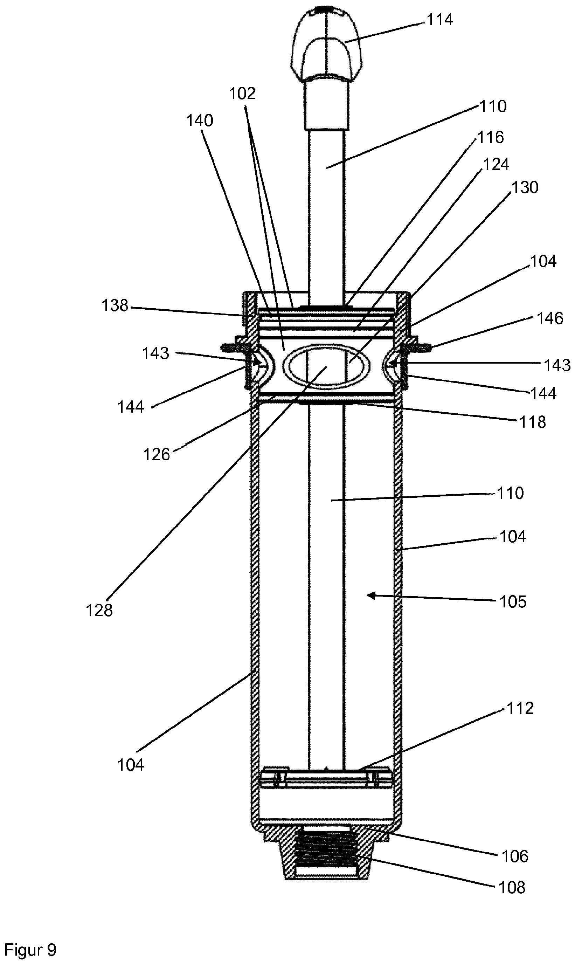

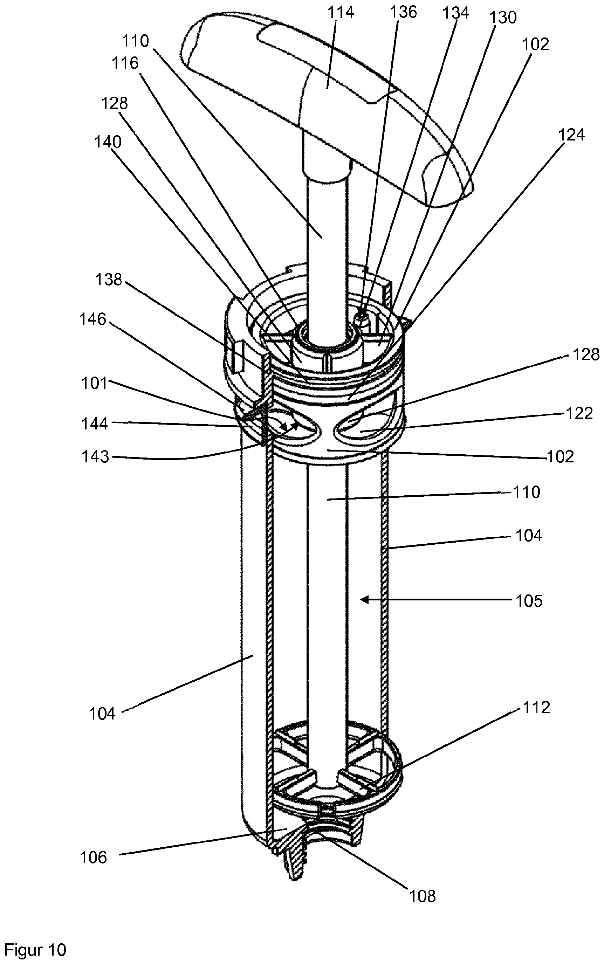

Die Figuren 5 bis 8 zeigen ein zweites alternatives Ausführungsbeispiel der vorliegenden Erfindung beziehungsweise ein zweites erfindungsgemäßes Vakuummischsystem, bei dem eine Durchführung 101 durch einen weitgehend zylindrischen Austragskolben 102 offen ist, und die Figuren 9 und 10 das gleiche alternative Ausführungsbeispiel der vorliegenden Erfindung beziehungsweise das zweite erfindungsgemäße Vakuummischsystem bei dem die Durchführung 101 durch den Austragskolben 102 geschlossen ist. Dabei zeigt Figur 5 eine perspektivische Außenansicht, Figur 6 eine perspektivische Teilschnittansicht, Figur 7 eine Querschnittansicht, Figur 8 eine Querschnittansicht eines Ausschnitts, Figur 9 eine seitliche Teilschnittansicht und Figur 10 eine perspektivische Teilschnittansicht des Vakuummischsystems.

Der Aufbau dieser alternativen Ausführungsform ähnelt im Wesentlichen dem Aufbau des ersten Ausführungsbeispiels.

Das Vakuummischsystem weist eine großenteils rohrförmige Kartusche 104 mit einem Innenraum 105 auf. In dem Innenraum 105 ist ein Zementpulver (nicht gezeigt) zur Herstellung eines Zementteigs aus zwei Komponenten enthalten. Die Kartusche 104 ist an der Vorderseite (in den Figuren 5 bis 7 und 9 und 10 unten und in Figur 8 nicht zu sehen) mit einer Platte 106 verschlossen, in der eine Öffnung mit einem Innengewinde 108 vorgesehen ist. An das Innengewinde 108 kann eine Basisplatte (nicht gezeigt) beziehungsweise ein Standfuß (nicht gezeigt) oder ein Austragsrohr (nicht gezeigt) zum Applizieren des fertig gemischten Zementteigs montiert werden. Über die Basisplatte beziehungsweise den Standfuß wird ein Flüssigkeitsbehälter (nicht gezeigt) mit einem flüssigen Monomer als zweiter flüssiger Komponente des Knochenzements mit der Kartusche 104 über eine Leitung (nicht gezeigt) verbunden. Dazu mündet die Leitung in einen Stutzen an der Basisplatte beziehungsweise dem Standfuß, wobei an dem Stutzen ein Außengewinde vorgesehen ist, so dass die Kartusche 104 mit dem Innengewinde 108 auf den Stutzen geschraubt werden kann beziehungsweise geschraubt ist, so dass die Leitung von dem Flüssigkeitsbehälter druckdicht über die Leitung mit der Kartusche 104 verbindbar oder verbunden ist. Ein Vakuummischsystem mit einem solchen Standfuß beziehungsweise einer solchen Basisplatte, einer solchen Leitung, einem solchen Stutzen und einem solchen Flüssigkeitsbehälter ist beispielsweise aus der DE 10 2009 031 178 B3 beziehungsweise der US 8,757,866 B2 bekannt, so dass für Details hierzu auf diese Patente verwiesen werden kann. Für die folgende Betrachtung sei angenommen, dass die Kartusche 104 an der Vorderseite zunächst nach außen abgeschlossen ist, indem die Kartusche 104 mit dem Innengewinde 108 auf einen solchen Standfuß beziehungsweise eine solche Basisplatte des Vakuummischsystems aufgeschraubt ist.

Auf der Rückseite (in den Figuren 5 bis 10 in Richtung oben) ist die Kartusche 104 durch den Austragskolben 102 verschlossen. Durch eine Durchführung im Austragskolben 102 erstreckt sich eine Stange 110, die an der Vorderseite im Innenraum 105 der Kartusche 104 in einem Mischelement 112 endet, das vier sich in Richtung der Wandung der Kartusche 104 radial ersteckenden Mischflügel aufweist. Die Stange 110 endet an der Rückseite (in den Figuren 5 bis 7 und 9 und 10 oben und in Figur 8 nicht zu sehen) in einem Handgriff 114, mit dem die Stange 110 manuell bewegt werden kann. Die Stange 110 ist um ihre Achse drehbar und in axialer Richtung (in Längsrichtung verschiebbar) in dem Austragskolben 102 gelagert. Hierzu sind in dem Austragskolben 102 zwei Lagerringe 116, 118 vorgesehen, die in Passung an der Stange 110 anliegen und die Stange 110 lagern.

Der untere Lagerring 118 dient auch der Abdichtung des Innenraums 105, um ein Austreten von Pulver oder Zementteig aus dem Innenraum 105 zu verhindern. Um den Innenraum 105 gasdicht und druckdicht abzudichten, ist zudem eine Dichtung 120 in Form eines O-Rings aus Gummi vorgesehen.

Durch die manuelle Drehbarkeit und axiale Verschiebbarkeit der Stange 110 kann der Inhalt des Innenraums 105 mit dem Mischelement 112 manuell durchmischt werden.

Die Durchführung 101 durch den Austragskolben 102 ist an der Vorderseite des Austragskolbens 102 mit einer Porenscheibe 122 abgedeckt. Die Porenscheibe 122 verhindert ein Eindringen von Zementpulver in die Durchführung 101 beziehungsweise ein Austreten von Zementpulver aus dem Vakuummischsystem.

Am Außenumfang des Austragskolbens 102 ist eine umlaufende Dichtung 124 in Form eines O-Rings aus Gummi vorgesehen, mit der der Zwischenraum zwischen dem Austragskolben 102 und der Innenwand der Kartusche 104 abgedichtet ist und damit der Innenraum 105 nach außen gasdicht und druckdicht abgeschlossen ist, wenn der Austragskolben 102 in die Kartusche 104 eingedrückt beziehungsweise eingesetzt ist, wie dies in den Figuren 5 bis 10 gezeigt ist.

An der Vorderseite weist der Austragskolben 102 eine umlaufende Abstreiflippe 126 auf, mit der beim Austragen eines fertig gemischten Knochenzements aus dem Innenraum 105 durch die Öffnung in der Platte 106 mit Hilfe des Austragskolbens 102 der Zementteig vollständig nach vorne getrieben wird, ohne dass sich der Zementteig an der Abstreiflippe 126 vorbei aus dem Innenraum 105 herausdrücken kann. Dazu ist die Abstreiflippe 126 verformt. Die Abstreiflippe 126 wird von ihrer Ausgangsform entsprechend verformt. Durch die dabei auftretende elastische Kraft wird der Innenraum 105 mit der Abstreiflippe 126 abgedichtet. Das gleiche Prinzip der Abdichtung, in dem Fall gegen die Stange 110, wird auch für den unteren Lagerring 118 angewendet.

Im Austragskolben 102 ist die Stange 110 durch eine Hülse 128 des Austragskolbens 102 geführt. Von der Hülse 128 erstrecken sich radial vier Streben 130 des Austragskolbens 102 in Richtung des Außenumfangs des Austragskolbens 102. Die Streben 130 dienen der mechanischen Stabilisierung und Formgebung des Austragskolbens 102 und der Lagerung der Porenscheibe 122. Rückseitig ist der Austragskolben 102 zwischen den Öffnungen in der Mantelfläche des Austragskolbens 102 für die Durchführung 101 und dem rückseitigen Ende des Austragskolbens 102 (in den Figuren 5 bis 10 oben) durch eine Platte 132 verschlossen. Die Platte 132 ist Teil des Austragskolbens 102 und mit diesem einteilig ausgeführt.

Eine Vakuumdurchführung 134 ist in der Platte 132 vorgesehen, die in einen Vakuumanschluss 136 in Form eines Stutzens 136 mündet. Auf den Vakuumanschluss 136 kann ein Schlauch (nicht gezeigt) aufgesteckt werden, der an eine Vakuumquelle (nicht gezeigt) anschließbar ist.

Im Innenumfang der Kartusche 104 ist eine umlaufende Feder 138 in Form eines Rings 138 vorgesehen, die als Rastmittel 138 zur Arretierung des Austragskolbens 102 in der Kartusche 104 vorgesehen ist. Als Gegenrastmittel 140 ist in dem Austragskolben 102 eine umlaufende Nuten 140 beziehungsweise eine umlaufende Vertiefung 140 vorgesehen, so dass der Austragskolben 102 bezüglich seiner Beweglichkeit in Längsrichtung in der Kartusche 104 arretierbar ist. Anstatt des Systems aus Nut 140 und Feder 138 könnten auch andere, beispielsweise manuell verformbare und damit einfacher lösbare Arretierungsmittel vorgesehen sein, ohne dass hierdurch die Erfindung eingeschränkt würde.

In der Wandung der Kartusche 104 ist wenigstens eine Öffnung 143 vorgesehen, die in der in den Figuren 5 bis 10 gezeigten Position des Austragskolbens 102 in Überdeckung mit der Öffnung in der Mantelfläche des Austragskolbens 102 liegt, die zur Durchführung 101 durch den Austragskolben 102 führt. Am Außenumfang der Kartusche 104 ist eine ringförmige Manschette 144 als Verschlusselement 144 vorgesehen. Diese Manschette 144 weist einen Innenumfang auf, der genauso groß oder geringfügig kleiner ist als der Außenumfang der Kartusche 104, so dass die Manschette 144 in Passung auf der Kartusche 104 aufsteckt. Mit Hilfe von Vorsprüngen 146 oder Griffen 146 lässt sich die Manschette 144 entlang der Zylinderachse der Kartusche 104 bewegen und dadurch die Öffnungen 143 in der Wandung der Kartusche 104 verschließen.

In die an der vorderen Platte 106 geschlossene Kartusche 104 wird zunächst das Knochenzementpulver eingefüllt. Dazu ist der Austragskolben 102 zunächst noch nicht in die Kartusche 104 eingesetzt. Anschließend wird der Austragskolben 102 in die Kartusche 104 eingesteckt, bis die Feder 138 mit der Nut 140 arretiert. Die Manschette 144 bedeckt dabei nicht die Öffnung 143 beziehungsweise Öffnungen 143. Diese geöffnete Position ist in den Figuren 5 bis 8 dargestellt. Durch die offene Durchführung 101 und die offenliegende Öffnung 143 kann die gesamte Kartusche 104 beziehungsweise das gesamte Vakuummischsystem in einer Kammer durch Evakuieren der Luft aus der Umgebung der Kartusche 104 beziehungsweise des Vakuummischsystems und durch Befüllen der Umgebung der Kartusche 104 beziehungsweise des Vakuummischsystems mit Ethylenoxid desinfiziert beziehungsweise sterilisiert werden.

Anschließend wird die Manschette 144 über die Öffnung 143 geschoben. Durch die Manschette 144 werden die Öffnungen 143 oder wird die Öffnung 143 in der Wandung der Kartusche 104 zu den Durchgängen 101 verschlossen. Durch die Dichtung 124 wird der Innenraum 105 bis auf die Vakuumdurchführung 136 druckdicht und gasdicht verschlossen, indem die Dichtung 124 an den Innenwänden der Kartusche 104 anliegt.

An den Vakuumanschluss 134 wird ein Schlauch angeschlossen, der mit einer Vakuumquelle verbunden ist. Durch die Vakuumdurchführung 136 wird die Luft aus dem Innenraum 105 herausgesaugt. Mit der Porenscheibe 122 wird verhindert, dass Pulver aus dem Innenraum 105 in das Vakuumsystem gelangen kann. Da der Innenraum 105 bis auf die an der Platte 106 beziehungsweise dem Innengewinde 108 angeschlossene Leitung und den Flüssigkeitsbehälter druckdicht ist, wird der Innenraum 105 evakuiert. Durch das Vakuum im Innenraum 105 wird die Manschette 144 auf die Öffnung 143 aufgedrückt beziehungsweise angesaugt und die Öffnung 143 dadurch effektiv verschlossen. Die Leitung und der Flüssigkeitsbehälter des Vakuummischsystems sind nach außen gasdicht und druckdicht geschlossen. Durch die Leitung wird das Monomer aus dem Flüssigkeitsbehälter in den Innenraum 105 gesaugt. Dort mischt es sich mit dem Zementpulver.

Das Zementpulver kann mit der Monomer-Flüssigkeit mit dem Mischelement 112 im Innenraum 105 unter Vakuum durch drehen und hineinstoßen und herausziehen der Stange 110 manuell gemischt werden. Nach erfolgter Mischung wird die Kartusche 104 von dem Standfuß beziehungsweise der Basisplatte abgeschraubt und auf das Innengewinde 108 wird ein Austragsrohr (nicht gezeigt) aufgeschraubt. Das Austragsrohr kann einen statischen Mischer enthalten. Die Stange 110 kann bis zum Anschlag aus dem Innenraum 105 herausgezogen werden, so dass das Mischelement 112 an der Vorderseite des Austragskolbens 102 anliegt. Die Stange 110 kann mit dem Handgriff 114 an einer Sollbruchstelle (nicht gezeigt) abgebrochen werden. Anschließend kann die Kartusche 104 in eine Auspressvorrichtung (nicht gezeigt) zum Vortreiben des Austragskolbens 102 in der Kartusche 104 eingesetzt werden, oder der Austragskolben 102 wird mit Hilfe eines Stößels (nicht gezeigt) vorgetrieben. Beispielsweise kann der Austragskolben 102 aus seiner arretierten Position entrastet werden und über das weiter anliegende Vakuum wird der Austragskolben 102 in der Kartusche 104 vorangetrieben, da außerhalb der Kartusche 104 nun kein Vakuum sondern Umgebungsdruck anliegt.

Durch das Vortreiben des Austragskolbens 102 wird der Zementteig aus dem Innenraum 105 der Kartusche 104 herausgetrieben und über das Austragsrohr appliziert.

Alle Teile des Vakuummischsystems sind aus Kunststoff durch Spritzguss gefertigt, wobei die Dichtungen 120, 124 bevorzugt aus Gummi oder einem anderen elastischen Kunststoff bestehen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101: Durchführung
- 2, 102: Austragskolben
- 4, 104: Kartusche
- 5, 105: Innenraum der Kartusche
- 6, 106: Platte
- 8, 108: Innengewinde
- 10, 110: Stange
- 12, 112: Mischelement
- 14, 114: Handgriff
- 16, 116: Lagerung / Lagerring
- 18, 118: Lagerung / Lagerring
- 20, 120: Dichtung / O-Ring
- 22, 122: Porenscheibe
- 24, 124: Dichtung / O-Ring
- 26, 126: Abstreiflippe
- 28, 128: Hülse
- 30, 130: Strebe
- 32, 132: Platte
- 34, 134: Vakuumanschluss / Stutzen
- 36, 136: Vakuumdurchführung
- 38, 138: Feder / Ring
- 40, 140: Nut / Vertiefung
- 42: Nut / Vertiefung
- 143: Öffnung in der Kartuschenwandung
- 144: Verschlusselement / Manschette
- 146: Vorsprung / Griff

## Patentansprüche

1. Vakuummischsystem zum Mischen von Polymethylmethacrylat-Knochenzement aufweisend
zumindest eine Kartusche (4, 104) mit einem evakuierbaren Innenraum (5, 105) zum Mischen des Knochenzements, wobei der Innenraum (5, 105) einen zylindrischen Hubraum umfasst,
ein Mischelement (12, 112), das im Innenraum (5, 105) der Kartusche (4, 104) beweglich angeordnet ist und das von außerhalb des Vakuummischsystems zum Mischen des Inhalts im Innenraum (5, 105) der Kartusche (4, 104) bedienbar ist und
einen Austragskolben (2, 102) mit einem zylindrischen Außenumfang, dessen erste Grundfläche eine Grundfläche des Innenraums (5, 105) der Kartusche (4, 104) begrenzt, der lösbar mit der Kartusche (4, 104) zu arretieren oder arretiert ist und der im gelösten Zustand beweglich im zylindrischen Bereich des Innenraums (5, 105) der Kartusche (4, 104) ist,
wobei in dem Austragskolben (2, 102) eine gasdurchlässige und für Partikel undurchlässige Durchführung (1, 101) angeordnet ist oder zwischen dem Austragskolben (2, 102) und der Innenwand des Innenraums (5, 105) eine Durchführung (1, 101) gebildet ist, wobei die Durchführung (1, 101) sich von einer Öffnung in der Mantelfläche des Austragskolbens (2, 102) bis zu einer Öffnung in der ersten Grundfläche des Austragskolbens (2, 102) erstreckt.

2. Vakuummischsystem nach Anspruch 1, **dadurch gekennzeichnet, dass**
der Austragskolben (2, 102) wenigstens eine umlaufende Dichtung (20, 120) aufweist, die den Innenraum (5, 105) der Kartusche (4, 104) nach außen abdichtet, wobei bevorzugt wenigstens eine umlaufende Dichtung (20, 120) zwischen einer zweiten Grundfläche des Austragskolbens (2, 102), die der ersten Grundfläche des Austragskolbens (2, 102) gegenüberliegt, und der Öffnung in der Mantelfläche des Austragskolbens (2, 102) angeordnet ist.

3. Vakuummischsystem nach Anspruch 1 oder 2, **dadurch gekennzeichnet, dass** eine der Mantelflächen der Kartuschenwandung der Kartusche (4, 104) eine Öffnung (143) aufweist, die mit der Öffnung in der Mantelfläche des Austragskolbens (2, 102) in einer geöffneten Position des Austragskolbens (2, 102) in Überdeckung liegt und durch die der Innenraum (5, 105) gasdurchlässig mit der Umgebung des Vakuummischsystems verbunden oder verbindbar ist.

4. Vakuummischsystem nach Anspruch 3, **dadurch gekennzeichnet, dass**
an der Außenwand der Kartusche (4, 104) ein Verschlusselement (144) angeordnet ist, mit dem die Öffnung (143) in der Wandung der Kartusche (4, 104) verschließbar ist, bevorzugt ein in axialer Richtung der Kartusche (4, 104) verschiebbares Verschlusselement (144) an der Außenwand der Kartusche (4, 104) angeordnet ist.

5. Vakuummischsystem nach Anspruch 4, **dadurch gekennzeichnet, dass**
das Verschlusselement (144) eine umlaufende Manschette (144) ist, die in Passung auf der Außenwand der Kartusche (4, 104) anliegt und in axialer Richtung der Kartusche (4, 104) verschiebbar ist, um die Öffnung (143) in der Wandung der Kartusche (4, 104) abzudecken und dadurch zu verschließen, wobei bevorzugt an der Manschette (144) wenigstens ein Griffstück (146) befestigt ist, das zum manuellen Verschieben der Manschette (144) auf der Außenwand der Kartusche (4, 104) vorgesehen ist.

6. Vakuummischsystem nach einem Ansprüche 1 oder 2, **dadurch gekennzeichnet, dass**
der Austragskolben (2, 102) in einer ersten arretierbaren Stellung aus der Kartusche (4, 104) herausragt, so dass die Öffnung in der Mantelfläche des Austragskolbens (2, 102) offen liegt und dass der Austragskolben (2, 102) in einer zweiten arretierbaren Stellung tiefer in dem Innenraum (5, 105) der Kartusche (4, 104) angeordnet ist, so dass die Öffnung in der Mantelfläche des Austragskolbens (2, 102) durch die Innenwand der Kartusche (4, 104) verschlossen ist.

7. Vakuummischsystem nach Anspruch 6, **dadurch gekennzeichnet, dass** zwischen der Öffnung in der Mantelfläche des Austragskolbens (2, 102) und der zweiten Grundfläche des Austragskolbens (2, 102), die der ersten Grundfläche des Austragskolbens (2, 102) gegenüberliegt, ein umlaufendes Dichtungselement (20, 120) angeordnet ist.

8. Vakuummischsystem nach einem der vorangehenden Ansprüche, **dadurch gekennzeichnet, dass**
in der Durchführung (1, 101) und/oder an der Öffnung zur Durchführung (1, 101) in der Mantelfläche des Austragskolbens (2, 102) und/oder an der Öffnung zur Durchführung (1, 101) in der ersten Grundfläche des Austragskolbens (2, 102) ein gasdurchlässiger Partikelfilter (22, 122), insbesondere ein Porenfilter (22, 122), angeordnet ist, wobei bevorzugt der gasdurchlässige Partikelfilter (22, 122) für Partikel mit einem Durchmesser von über 1 µm undurchlässig ist, besonders bevorzugt für Partikel mit einem Durchmesser von über 5 µm undurchlässig ist.

9. Vakuummischsystem nach einem der vorangehenden Ansprüche, **dadurch gekennzeichnet, dass**
der Austragskolben (2, 102) an seiner aus dem Inneren der Kartusche (4, 104) weisenden Seite bis auf eine Vakuumdurchführung (36, 136) gasdicht ist.

10. Vakuummischsystem nach einem der vorangehenden Ansprüche, **dadurch gekennzeichnet, dass**
der Austragskolben (2, 102) an der ersten Grundfläche eine gasdurchlässige Porenscheibe (22, 122) aufweist, die durch eine Verrippung gestützt wird.

11. Vakuummischsystem nach einem der vorangehenden Ansprüche, **dadurch gekennzeichnet, dass**
der Austragskolben (2, 102) axial in die Kartusche (4, 104) eindrückbar ist, um einen aus einem Knochenzementpulver und einer Monomerflüssigkeit gemischten Knochenzementteig durch eine Austragsöffnung an einem dem Austragskolben (2, 102) gegenüberliegenden Ende der Kartusche (4, 104) auszutragen, wobei bevorzugt der Austragskolben (2, 102) nach dem Lösen einer Arretierung axial in die Kartusche (4, 104) eindrückbar ist.

12. Vakuummischsystem nach einem der vorangehenden Ansprüche, **dadurch gekennzeichnet, dass**
eine Austragsöffnung der Kartusche (4, 104) ein Anschlussmitteln (8, 108), insbesondere ein Anschlussgewinde (8, 108), aufweist.

13. Vakuummischsystem nach einem der vorangehenden Ansprüche, **dadurch gekennzeichnet, dass**
das Mischelement (12, 112) an einer Stange (10, 110) angeordnet ist, die durch einen gasdichten Durchgang ins Innere der Kartusche (4, 104) geführt ist, und sich das Mischelement (12, 112) durch hineinschieben und herausziehen aus der Kartusche (4, 104) und durch Drehen in der Kartusche (4, 104) bewegen lässt, wobei bevorzugt die Stange (10, 110) eine Sollbruchstelle aufweist, an der die Stange (10, 110), wenn sie aus der Kartusche (4, 104) herausgezogen ist, nahe dem Durchgang abzubrechen ist.

14. Vakuummischsystem nach einem der vorangehenden Ansprüche, **dadurch gekennzeichnet, dass**
das Vakuummischsystem einen Vorratsbehälter für eine Monomerflüssigkeit aufweist, insbesondere einen Glasbehälter für eine Monomerflüssigkeit aufweist, in der Kartusche (4, 104) ein Zementpulver enthalten ist, wobei ein Öffnungselement zur Öffnung des Vorratsbehälters vorgesehen ist und die Kartusche (4, 104) über eine Leitung mit dem geöffneten Vorratsbehälter verbunden oder verbindbar ist.

15. Vakuummischsystem nach Anspruch 14, **dadurch gekennzeichnet, dass**
das Vakuummischsystem ein Basiselement aufweist, wobei das Basiselement die Kartusche (4, 104), den Vorratsbehälter und das Öffnungselement lagert.

16. Vakuummischsystem nach Anspruch 15, **dadurch gekennzeichnet, dass**
das Basiselement ein Koppelmittel aufweist für eine kraft- und/oder formschlüssige Verbindung mit der Kartusche (4, 104), insbesondere mit dem Anschlussmittel (8, 108) an der Austragsöffnung der Kartusche (4, 104).

17. Vakuummischsystem nach einem der Ansprüche 14 bis 16, **dadurch gekennzeichnet, dass**
in der Leitung ein Ventilelement angeordnet ist, das den Ausfluss der Monomerflüssigkeit aus dem Vorratsbehälter in die Kartusche (4, 104) steuert und/oder auslöst.

18. Vakuummischsystem nach einem der Ansprüche 14 bis 16, **dadurch gekennzeichnet, dass**
an der Kartusche (4, 104) ein Rastmittel (38, 138) und an dem Austragskolben (2, 102) mindestens ein Gegenrastmittel (40, 42, 140) vorgesehen ist, wobei der Austragskolben (2, 102) mit dem Rastmittel (38, 138) in der Kartusche (4, 104) in einer Position lösbar arretiert oder lösbar arretierbar ist, oder in zumindest zwei Positionen lösbar arretierbar ist, wobei die Durchführung (1, 101) in einer ersten arretierten Position geöffnet ist und in einer zweiten arretierten Position gasdicht verschlossen ist.

19. Verfahren zur Vermischung von Polymethylmethacrylat-Knochenzement in einer Kartusche (4, 104) eines Vakuummischsystems nach einem der vorangehenden Ansprüche, **dadurch gekennzeichnet, dass**
ein durch einen Austragskolben (2, 102) verschlossener Innenraum (5, 105) einer Kartusche (4, 104) durch eine gasdurchlässigen Durchführung (1, 101) im oder am Austragskolben (2, 102) offenliegt, wobei Gas aus dem Innenraum (5, 105) evakuiert wird und der Innenraum (5, 105) mit einem sterilisierenden Gas gefüllt wird, wobei danach die Durchführung (1, 101) durch Verschieben des Austragskolbens (2, 102) verschlossen wird oder durch Bedienen eines Verschlusselements (144) am Außenumfang der Kartusche (4, 104) verschlossen wird, danach der Innenraum (5, 105) über einen Vakuumdurchführung (36, 136) evakuiert wird und danach Ausgangskomponenten des Knochenzements mit einem Mischelement (12, 112) im Innenraum (5, 105) der Kartusche (4, 104) unter Vakuum gemischt werden.

20. Verfahren nach Anspruch 19, **dadurch gekennzeichnet, dass**
der Austragskolben (2, 102) nach dem Einsetzen des Austragskolbens (2, 102) in die Kartusche (4, 104) mit der Kartusche (4, 104) arretiert wird und/oder der Austragskolben (2, 102) nach dem Verschieben des Austragskolbens (2, 102) arretiert wird, so dass er nicht durch Einwirkung des Unterdrucks in die Kartusche (4, 104) gezogen wird, und die Arretierung nach dem Mischen des Knochenzements gelöst wird und der gemischte Knochenzementteig aus der Kartusche (4, 104) durch Vortreiben des Austragskolbens (2, 102) im Innenraum (5, 105) der Kartusche (4, 104) durch eine gegenüberliegende Austragsöffnung ausgetrieben wird.

21. Verfahren nach Anspruch 19 oder 20, **dadurch gekennzeichnet, dass**
ein Zementpulver in dem Innenraum (5, 105) der Kartusche (4, 104) enthalten ist, eine Monomerflüssigkeit in den Innenraum (5, 105) der Kartusche (4, 104) eingeleitet wird, bevorzugt mit dem Unterdruck in dem Innenraum (5, 105) der Kartusche (4, 104) eingesaugt wird, und die Monomerflüssigkeit mit dem Zementpulver in dem evakuierten Innenraum (5, 105) der Kartusche (4, 104) gemischt wird.

## Claims

1. A vacuum mixing system for the mixing of polymethylmethacrylate bone cement, comprising
at least one cartridge (4, 104) with an evacuable internal space (5, 105) for the mixing of the bone cement, whereby the internal space (5, 105) comprises a cylindrical swept volume;
a mixing element (12, 112) that is arranged in the internal space (5, 105) of the cartridge (4, 104) such as to be mobile and that can be operated from outside of the vacuum mixing system in order to mix the content in the internal space (5, 105) of the cartridge (4, 104); and
a dispensing plunger (2, 102) with a cylindrical external circumference, whose first base surface borders a base surface of the internal space (5, 105) of the cartridge (4, 104), that can be locked or is locked detachably to the cartridge (4, 104), and, in detached condition, is mobile in the cylindrical section of the internal space (5, 105) of the cartridge (4, 104);
whereby a gas-permeable and particle-impermeable feed-through (1, 101) is arranged in the dispensing plunger (2, 102), or a feed-through (1, 101) is formed between the dispensing plunger (2, 102) and the internal wall of the internal space (5, 105), whereby the feed-through (1, 101) extends from an opening in the jacket surface of the dispensing plunger (2, 102) to an opening in the first base surface of the dispensing plunger (2, 102).

2. The vacuum mixing system according to claim 1, **characterised in that**
the dispensing plunger (2, 102) comprises at least one all-round seal (20, 120) that seals the internal space (5, 105) of the cartridge (4, 104) with respect to the outside, whereby preferably at least one all-round seal (20, 120) is arranged between a second base surface of the dispensing plunger (2, 102), which is situated opposite from the first base surface of the dispensing plunger (2, 102), and the opening in the jacket surface of the dispensing plunger (2, 102).

3. The vacuum mixing system according to claim 1 or 2, **characterised in that** one of the jacket surfaces of the cartridge wall of the cartridge (4, 104) comprises an opening (143) that coincides with the opening in the jacket surface of the dispensing plunger (2, 102) in an opened position of the dispensing plunger (2, 102), and by means of which the internal space (5, 105) can be connected or is connected in gas-permeable manner to the surroundings of the vacuum mixing system.

4. The vacuum mixing system according to claim 3, **characterised in that**
a closure element (144) is arranged on the external wall of the cartridge (4, 104) and can be used to close the opening (143) in the wall of the cartridge (4, 104), preferably a closure element (144) that can be shifted in axial direction of the cartridge (4, 104) is arranged on the external wall of the cartridge (4, 104).

5. The vacuum mixing system according to claim 4, **characterised in that**
the closure element (144) is an all-round cuff (144) that touches in a tight fit against the external wall of the cartridge (4, 104) and can be shifted in axial direction of the cartridge (4, 104) in order to cover and thus close the opening (143) in the wall of the cartridge (4, 104), whereby it is preferred to have at least one handle part (146) attached to the cuff (144) that is intended for manual shifting of the cuff (144) on the external wall of the cartridge (4, 104).

6. The vacuum mixing system according to either one of the claims 1 or 2, **characterised in that**
the dispensing plunger (2, 102) in a first lockable position projects from the cartridge (4, 104) such that the opening in the jacket surface of the dispensing plunger (2, 102) is open, and **in that** the dispensing plunger (2, 102) in a second lockable position is arranged more deeply in the internal space (5, 105) of the cartridge (4, 104) such that the opening in the jacket surface of the dispensing plunger (2, 102) is closed by the internal wall of the cartridge (4, 104).

7. The vacuum mixing system according to claim 6, **characterised in that** an all-round sealing element (20, 120) is arranged between the opening in the jacket surface of the dispensing plunger (2, 102) and the second base surface of the dispensing plunger (2, 102), which is situated opposite from the first base surface of the dispensing plunger (2, 102).

8. The vacuum mixing system according to any one of the preceding claims, **characterised in that**
a gas-permeable particle filter (22, 122), in particular a pore filter (22, 122) is arranged in the feed-through (1, 101) and/or at the opening to the feed-through (1, 101) in the jacket surface of the dispensing plunger (2, 102) and/or at the opening to the feed-through (1, 101) in the first base surface of the dispensing plunger (2, 102), whereby the gas-permeable particle filter (22, 122) preferably is impermeable to particles with a diameter of more than 1 µm, particularly preferably is impermeable to particles with a diameter of more than 5 µm.

9. The vacuum mixing system according to any one of the preceding claims, **characterised in that**
the dispensing plunger (2, 102) is gas-tight, with the exception of a vacuum feed-through (36, 136), on its side facing out of the inside of the cartridge (4, 104).

10. The vacuum mixing system according to any one of the preceding claims, **characterised in that**
the dispensing plunger (2, 102) comprises, on the first base surface, a gas-permeable pore disk (22, 122) that is supported by a ribbing.

11. The vacuum mixing system according to any one of the preceding claims, **characterised in that**
the dispensing plunger (2, 102) can be axially pushed into the cartridge (4, 104) in order to dispense a bone cement dough, which has been produced by mixing a bone cement powder and a monomer liquid, through a dispensing opening at an end of the cartridge (4, 104), which is situated opposite from the dispensing plunger (2, 102), whereby the dispensing plunger (2, 102) preferably can be axially pushed into the cartridge (4, 104) after detaching a lock.

12. The vacuum mixing system according to any one of the preceding claims, **characterised in that**
a dispensing opening of the cartridge (4, 104) comprises connecting means (8, 108), in particular a connecting thread (8, 108).

13. The vacuum mixing system according to any one of the preceding claims, **characterised in that**
the mixing element (12, 112) is arranged on a rod (10, 110) that is guided through a gas-tight passage into the inside of the cartridge (4, 104), and **in that** the mixing element (12, 112) can be moved by pushing and pulling it into and out of the cartridge (4, 104) and by rotating it in the cartridge (4, 104), whereby the rod (10, 110) preferably comprises a predetermined breakage site at which the rod (10, 110) can be broken off near the passage, when it is pulled out of the cartridge (4, 104).

14. The vacuum mixing system according to any one of the preceding claims, **characterised in that**
the vacuum mixing system comprises a reservoir container for a monomer liquid, in particular comprises a glass container for a monomer liquid, **in that** the cartridge (4, 104) contains a cement powder, whereby an opening element for opening the reservoir container is provided and the cartridge (4, 104) is connected or can be connected to the opened reservoir container by means of a conduit.

15. The vacuum mixing system according to claim 14, **characterised in that** the vacuum mixing system comprises a basic element, whereby the base element supports the cartridge (4, 104), the reservoir container, and the opening element.

16. The vacuum mixing system according to claim 15, **characterised in that**
the basic element comprises a coupling means for a force-locked and/or form-fitting connection to the cartridge (4, 104), in particular to the connecting means (8, 108) at the dispensing opening of the cartridge (4, 104).

17. The vacuum mixing system according to any one of the claims 14 to 16, **characterised in that**
the conduit has a valve element arranged in it that controls and/or triggers the discharge of the monomer liquid from the reservoir container into the cartridge (4, 104).

18. The vacuum mixing system according to any one of the claims 14 to 16, **characterised in that**
a snap-in means (38, 138) is provided on the cartridge (4, 104) and at least one opposite snap-in means (40, 42, 140) is provided on the dispensing plunger (2, 102), whereby the dispensing plunger (2, 102) is locked detachably or can be locked detachably in the cartridge (4, 104) in one position, or can be locked detachably in at least two positions, whereby the feed-through (1, 101) is opened in a first locked position and is closed in gas-tight manner in a second locked position.

19. A method for the mixing of polymethylmethacrylate bone cement in a cartridge (4, 104) of a vacuum mixing system according to any one of the preceding claims, **characterised in that**
an internal space (5, 105) of a cartridge (4, 104) that is closed by a dispensing plunger (2, 102) is laid open by means of a gas-permeable feed-through (1, 101) in or on the dispensing plunger (2, 102), whereby gas is being evacuated from the internal space (5, 105) and the internal space (5, 105) is filled with a sterilising gas, whereby the feed-through (1, 101) is subsequently being closed through shifting the dispensing plunger (2, 102) or is being closed by operating a closure element (144) on the external circumference of the cartridge (4, 104), with the internal space (5, 105) subsequently being evacuated via a vacuum feed-through (36, 136), and starting components of the bone cement subsequently being mixed in a vacuum in the internal space (5, 105) of the cartridge (4, 104) by means of a mixing element (12, 112).

20. The method according to claim 19, **characterised in that**
the dispensing plunger (2, 102) is being locked to the cartridge (4, 104) after the dispensing plunger (2, 102) is inserted into the cartridge (4, 104) and/or the dispensing plunger (2, 102) being locked after the dispensing plunger (2, 102) is shifted such that it is not drawn into the cartridge (4, 104) through the effect of the negative pressure, and the locking being detached after the bone cement is mixed and the mixed bone cement dough being expelled from the cartridge (4, 104) through an opposite dispensing opening by propelling the dispensing plunger (2, 102) in the internal space (5, 105) of the cartridge (4, 104).

21. The method according to claim 19 or 20, **characterised in that**
the internal space (5, 105) of the cartridge (4, 104) contains a cement powder, a monomer liquid is supplied into the internal space (5, 105) of the cartridge (4, 104), preferably is aspirated through the negative pressure in the internal space (5, 105) of the cartridge (4, 104), and the monomer liquid is mixed with the cement powder in the evacuated internal space (5, 105) of the cartridge (4, 104).

## Revendications

1. Système de mélange sous vide pour le mélange de ciment osseux en polyméthacrylate de méthyle présentant
au moins une cartouche (4, 104) avec un espace interne pouvant être évacué (5, 105) pour le mélange du ciment osseux, dans lequel l'espace interne (5, 105) comprend un espace de course cylindrique,
un élément de mélange (12, 112) qui est disposé de manière mobile dans l'espace interne (5, 105) de la cartouche (4, 104) et qui peut être manié de l'extérieur du système de mélange sous vide pour le mélange du contenu dans l'espace interne (5, 105) de la cartouche (4, 104), et
un piston d'extraction (2, 102) avec une périphérie externe cylindrique dont la première face de base délimite une face de base de l'espace interne (5, 105) de la cartouche (4, 104), qui est à bloquer ou bloqué de manière amovible avec la cartouche (4, 104) et qui est mobile dans l'état desserré dans la région cylindrique de l'espace interne (5, 105) de la cartouche (4, 104),
dans lequel une traversée (1, 101) perméable au gaz et imperméable pour des particules est disposée dans le piston d'extraction (2, 102) ou une traversée (1, 101) est formée entre le piston d'extraction (2, 102) et la paroi interne de l'espace interne (5, 105), dans lequel la traversée (1, 101) s'étend d'une ouverture dans la face d'enveloppe du piston d'extraction (2, 102) à une ouverture dans la première face de base du piston d'extraction (2, 102).

2. Système de mélange sous vide selon la revendication 1, **caractérisé en ce que**
le piston d'extraction (2, 102) présente au moins un joint périphérique (20, 120) qui étanchéifie l'espace interne (5, 105) de la cartouche (4, 104) vers l'extérieur, dans lequel au moins un joint périphérique (20, 120) est de préférence disposé entre une seconde face de base du piston d'extraction (2, 102) qui est opposée à la première face de base du piston d'extraction (2, 102) et l'ouverture dans la face d'enveloppe du piston d'extraction (2, 102).

3. Système de mélange sous vide selon la revendication 1 ou 2, **caractérisé en ce que**
une des faces d'enveloppe de la paroi de cartouche de la cartouche (4, 104) présente une ouverture (143) qui se situe en superposition avec l'ouverture dans la face d'enveloppe du piston d'extraction (2, 102) dans une position ouverte du piston d'extraction (2, 102) et à travers laquelle l'espace interne (5, 105) est relié ou peut être relié de manière perméable au gaz à l'environnement du système de mélange sous vide.

4. Système de mélange sous vide selon la revendication 3, **caractérisé en ce que**
un élément de fermeture (144) avec lequel l'ouverture (143) peut être fermée dans la paroi de la cartouche (4, 104) est disposé sur la paroi externe de la cartouche (4, 104), un élément de fermeture (144) pouvant être fermé dans la direction axiale de la cartouche (4, 104) est de préférence disposé sur la paroi externe de la cartouche (4, 104).

5. Système de mélange sous vide selon la revendication 4, **caractérisé en ce que**
l'élément de fermeture (144) est une manchette périphérique (144) qui repose en ajustement sur la paroi externe de la cartouche (4, 104) et peut être coulissée dans la direction axiale de la cartouche (4, 104) pour recouvrir et pour fermer de ce fait l'ouverture (143) dans la paroi de la cartouche (4, 104), dans lequel au moins une pièce de poignée (146) qui est prévue pour le coulissement manuel de la manchette (144) sur la paroi externe de la cartouche (4, 104) est fixée à la manchette (144).

6. Système de mélange sous vide selon une des revendications 1 ou 2, **caractérisé en ce que**
le piston d'extraction (2, 102) dépasse de la cartouche (4, 104) dans une première position pouvant être bloquée de sorte que l'ouverture dans la face d'enveloppe du piston d'extraction (2, 102) se situe de manière ouverte et que le piston d'extraction (2, 102) est disposé de manière plus profonde dans l'espace interne (5, 105) de la cartouche (4, 104) dans une seconde position pouvant être bloquée de sorte que l'ouverture dans la face d'enveloppe du piston d'extraction (2, 102) est fermée par la paroi interne de la cartouche (4, 104).

7. Système de mélange sous vide selon la revendication 6, **caractérisé en ce que**
un élément d'étanchéité périphérique (20, 120) est disposé entre l'ouverture dans la face d'enveloppe du piston d'extraction (2, 102) et la seconde face de base du piston d'extraction (2, 102) qui est opposée à la première face de base du piston d'extraction (2, 102).

8. Système de mélange sous vide selon une des revendications précédentes, **caractérisé en ce que**
un filtre à particules perméable au gaz (22, 122), notamment un filtre à pores (22, 122), est disposé dans la traversée (1, 101) et/ou sur l'ouverture vers la traversée (1, 101) dans la face d'enveloppe du piston d'extraction (2, 102) et/ou sur l'ouverture vers la traversée (1, 101) dans la première face de base du piston d'extraction (2, 102), dans lequel le filtre à particules perméable au gaz (22, 122) est de préférence imperméable pour des particules avec un diamètre de plus de 1 µm, est de manière particulièrement préférée imperméable pour des particules avec un diamètre de plus de 5 µm.

9. Système de mélange sous vide selon une des revendications précédentes, **caractérisé en ce que**
le piston d'extraction (2, 102) est étanche au gaz sur son côté tourné hors de l'intérieur de la cartouche (4, 104) à l'exception d'une traversée sous vide (36, 136).

10. Système de mélange sous vide selon une des revendications précédentes, **caractérisé en ce que**
le piston d'extraction (2, 102) présente sur la première face de base un disque à pores perméable au gaz (22, 122) qui est soutenu par un nervurage.

11. Système de mélange sous vide selon une des revendications précédentes, **caractérisé en ce que**
le piston d'extraction (2, 102) peut être enfoncé axialement dans la cartouche (4, 104) pour extraire une pâte de ciment osseux mélangée à partir d'une poudre de ciment osseux et d'un fluide monomère à travers une ouverture d'extraction à une extrémité de la cartouche (4, 104) opposée au piston d'extraction (2, 102), dans lequel le piston d'extraction (2, 102) peut de préférence être enfoncé axialement dans la cartouche (4, 104) après le desserrage d'un blocage.

12. Système de mélange sous vide selon une des revendications précédentes, **caractérisé en ce que**
une ouverture d'extraction de la cartouche (4, 104) présente un moyen de raccordement (8, 108), notamment un filetage de raccordement (8, 108).

13. Système de mélange sous vide selon une des revendications précédentes, **caractérisé en ce que**
l'élément de mélange (12, 112) est disposé sur une tige (10, 110) qui est guidée à travers un passage étanche au gaz dans l'intérieur de la cartouche (4, 104) et fait se déplacer l'élément de mélange (12, 112) en le rentrant et le sortant de la cartouche (4, 104) et en le faisant tourner dans la cartouche (4, 104), dans lequel la tige (10, 110) présente de préférence un point destiné à la rupture au niveau duquel la tige (10, 110) est à rompre à proximité du passage lorsqu'elle est sortie de la cartouche (4, 104).

14. Système de mélange sous vide selon une des revendications précédentes, **caractérisé en ce que**
le système de mélange sous vide présente un réservoir pour un fluide monomère, présente notamment un récipient en verre pour un fluide monomère, une poudre de ciment est contenue dans la cartouche (4, 104), dans lequel un élément d'ouverture est prévu pour l'ouverture du réservoir et la cartouche (4, 104) est connectée ou peut être connectée au réservoir ouvert par le biais d'une conduite.

15. Système de mélange sous vide selon la revendication 14, **caractérisé en ce que**
le système de mélange sous vide présente un élément de base, dans lequel l'élément de base stocke la cartouche (4, 104), le réservoir et l'élément d'ouverture.

16. Système de mélange sous vide selon la revendication 15, **caractérisé en ce que**
l'élément de base présente un moyen d'accouplement pour une connexion par friction et/ou conjugaison de formes avec la cartouche (4, 104), notamment avec le moyen de raccordement (8, 108) sur l'ouverture d'extraction de la cartouche (4, 104).

17. Système de mélange sous vide selon une des revendications 14 à 16, **caractérisé en ce que**
un élément de soupape qui commande et/ou déclenche l'écoulement du fluide monomère du réservoir dans la cartouche (4, 104) est disposé dans la conduite.

18. Système de mélange sous vide selon une des revendications 14 à 16, **caractérisé en ce que**
un moyen d'encliquetage (38, 138) est prévu sur la cartouche (4, 104) et au moins un moyen d'encliquetage conjugué (40, 42, 140) est prévu sur le piston d'extraction (2, 102), dans lequel le piston d'extraction (2, 102) est bloqué de manière amovible ou peut être bloqué de manière amovible dans une position avec le moyen d'encliquetage (38, 138) dans la cartouche (4, 104), ou peut être bloqué de manière amovible dans au moins deux positions, dans lequel la traversée (1, 101) est ouverte dans une première position bloquée et est fermée de manière étanche au gaz dans une seconde position bloquée.

19. Procédé de mélange de ciment osseux en polyméthacrylate de méthyle dans une cartouche (4, 104) d'un système de mélange sous vide selon une des revendications précédentes, **caractérisé en ce que**
un espace interne (5, 105) fermé par un piston d'extraction (2, 102) se situe de manière ouverte à travers une traversée perméable au gaz (1, 101) dans ou sur le piston d'extraction (2, 102), dans lequel du gaz est évacué de l'espace interne (5, 105) et l'espace interne (5, 105) est rempli d'un gaz stérilisant, dans lequel la traversée (1, 101) est ensuite fermée par coulissement du piston d'extraction (2, 102) ou est fermée par maniement d'un élément de fermeture (144) à la périphérie externe de la cartouche (4, 104), l'espace interne (5, 105) est ensuite évacué par le biais d'une traversée sous vide (36, 136) et des composants de départ du ciment osseux sont ensuite mélangés sous vide avec un élément de mélange (12, 112) dans l'espace interne (5, 105) de la cartouche (4, 104).

20. Procédé selon la revendication 19, **caractérisé en ce que**
le piston d'extraction (2, 102) est bloqué avec la cartouche (4, 104) après l'insertion du piston d'extraction (2, 102) dans la cartouche (4, 104) et/ou le piston d'extraction (2, 102) est bloqué après le coulissement du piston d'extraction (2, 102) de sorte qu'il n'est pas tiré par l'action de la dépression dans la cartouche (4, 104), et le blocage est desserré après le mélange du ciment osseux et la pâte de ciment osseux mélangée est expulsée de la cartouche (4, 104) par avancée du piston d'extraction (2, 102) dans l'espace interne (5, 105) de la cartouche (4, 104) à travers une ouverture d'extraction opposée.

21. Procédé selon la revendication 19 ou 20, **caractérisé en ce que**
une poudre de ciment est contenue dans l'espace interne (5, 105) de la cartouche (4, 104), un fluide monomère est introduit dans l'espace interne (5, 105) de la cartouche (4, 104), est de préférence aspiré avec la dépression dans l'espace interne (5, 105) de la cartouche (4, 104), et le fluide monomère est mélangé avec la poudre de ciment dans l'espace interne évacué (5, 105) de la cartouche (4, 104).
